(19) **Europäisches Patentamt**
**European Patent Office**
**Office européen des brevets**

(11) **EP 3 199 201 A1**

(12) **EUROPEAN PATENT APPLICATION**

(43) Date of publication:
**02.08.2017 Bulletin 2017/31**

(21) Application number: **17158902.1**

(22) Date of filing: **17.09.2012**

(51) Int Cl.:
*A61N 1/18* (2006.01)     *A61N 1/40* (2006.01)
*A61B 18/04* (2006.01)     *H05H 1/24* (2006.01)
*A61N 1/04* (2006.01)     *A61N 1/32* (2006.01)
*A61N 1/44* (2006.01)     *A61N 5/06* (2006.01)
*A61N 1/06* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.09.2011 US 201161535986 P**
**09.01.2012 US 201261584399 P**
**02.04.2012 PCT/US2012/031923**

(62) Document number(s) of the earlier application(s) in accordance with Art. 76 EPC:
**12831818.5 / 2 760 536**

(71) Applicant: **Moe Medical Devices LLC**
**New Rochelle NY 10804 (US)**

(72) Inventors:
• ZEMEL, Marc
  New Rochelle, NY 10804 (US)
• FRIEDMAN, Gennady
  Richboro, PA 18954 (US)

(74) Representative: **Maschio, Antonio**
**Maschio & Soames IP Limited**
**30 Carlton Crescent**
**Southampton SO15 2EW (GB)**

Remarks:
This application was filed on 02-03-2017 as a divisional application to the application mentioned under INID code 62.

(54) **SYSTEMS FOR ELECTRIC FIELD AND/OR PLASMA-ASSISTED ONYCHOMYCOSIS TREATMENT**

(57)     A system for applying a plasma discharge and/or electric field, comprising: a controller (4); an electrode (3) adapted to be placed proximate an anatomical region of interest, the electrode (3) having an array of conductors; and a power supply in electrical communication with the electrode (3) and controller (4), the power supply being adapted and configured to apply power to the electrode (3), characterized in that the array of conductors are adapted and configured to be selectively activated and deactivated by the controller (4), the power supply being adapted and configured to apply power to the electrode (3) to generate a moving plasma over time in the anatomical region of interest by activating a plurality of conductors in the array of conductors.

**EP 3 199 201 A1**

**Description**

CROSS-REFERENCE TO RELATED APPLICATIONS

This application claims the benefit of priority of International Application No. PCT/US2012/31923, filed April 2, 2012, U.S. Provisional Patent Application Serial No. 61/535,986, filed September 17, 2011 and U.S. Provisional Patent Application Serial No. 61/584,399, filed January 9, 2012. The disclosure of each of the aforementioned patent applications is incorporated by reference herein in its entirety.

BACKGROUND

Field of the Disclosure

The present disclosure relates to methods and systems for treating onychomycosis and other pathogenic infections of the nail. Particularly, the present disclosure is directed to the treatment of onychomycosis and other pathogenic infections of the nail in a manner that is assisted and/or enhanced by use of plasma and/or the application of strong electric fields.

Description of Related Art

Onychomycosis is a particularly difficult condition to treat due to the fact that the fungus often lives underneath or within the nail. The nail is mainly comprised of hard keratin, which makes it difficult to penetrate. A variety of techniques are known in the art for treating onychomycosis. These include topical drugs, systemic drugs, electrical heating, light-based heating, and ultraviolet light. Each of these treatments suffers from one or more shortcomings as described below:

1. Topical Drugs - are effective at killing the underlying fungal infection (such as *T. rubrum*), but have difficulty penetrating the nail. Common examples include ciclopirox, amorolfine, and terbinafine. Dosing cycles are also long - they can run from 6 to 18 months.
2. Systemic drugs - can also be effective at killing the underlying fungal infection, but have several potential side effects (such as liver failure) and require relatively long dosing cycles (daily pills up to 6 months). Common examples include terbinafine and itraconazole.
3. Electrical and light-based heating - various approaches have been attempted. However, most involve attempting to provide the heat required to kill the pathogen while preserving the underlying tissue. These attempts have proved difficult to implement in practice. Examples include Nomir Medical Technologies and PathoLase.
4. Ultraviolet Light - Keraderm, a startup company, has attempted to use UVC (light ranging from 200 to 280 nm) using a xenon flash lamp to kill the underlying infection. The nail will transmit some amount of UVC, but the exposure levels can be significant ($120 \text{ mJ/cm}^2$) and some dermatologists have expressed concern about causing skin cancer in the underlying nail bed, which would be quite difficult to treat.

The present disclosure presents improvements on the state of the art as set forth hereinbelow.

SUMMARY OF THE DISCLOSURE

The purpose and advantages of the present disclosure will be set forth in and become apparent from the description that follows. Additional advantages of the disclosed embodiments will be realized and attained by the methods and systems particularly pointed out in the written description hereof, as well as from the appended drawings.
To achieve these and other advantages and in accordance with the purpose of the disclosure, as embodied herein, the disclosure includes a variety of nail treatment methods that have been developed using the application of plasma and/or the application of electric fields. The infected nail and nail bed are porous structures. The pores are primarily created by the pathogenic micro-organisms that consume the nail (keratin) and nail bed material, creating microchannels similar to how earthworms burrow through soil. In many of the embodiments described herein, a high electric field is created within the nail and nail bed. When the electric field exceeds the air/gas breakdown field, plasma will be created within the pores of the nail bed and nail. Applicant also believes that the application of a high electric field in and of itself is of significant therapeutic benefit. The plasma within pores of the nail and nail bed can be sustained in the same way as the plasma in dielectric barrier discharges by pulsing or otherwise time varying the electric field. The electric field and the plasma created within the pores of the nail bed and the nail are two of the key agents that kill or slow down the growth of the infecting micro-organisms directly or indirectly by generating various chemically active species within micro-organism bodies and in the medium surrounding them. The direct effect of electric field on micro-organisms can include the effect of electroporation. Electroporation can inactivate (kill or slow the growth) of various micro-organisms including bacteria and fungi. Plasma can also affect micro-organism directly through the presence of charges, plasma generated electric field or through short penetration UV radiation generated in plasma. Estimated treatment time during which electric field and/or plasma within the nail and the nail bed occurs is preferably at least a tenth of a second and preferably no more than 1 hour, and in any desired time increment therebetween in increments of minutes, seconds or fractions of a second (e.g., 0.1, 0.01, 0.01, 0.001 seconds), as desired.

An electric field that may or may not (as desired) cause generation of plasma within the pores of the nail and nail bed can, in turn, be created by a multitude of different embodiments by applying high voltage to the surface of the nail. In order to apply the high voltage to the surface of the nail, the following techniques can be used. For example, a highly polarizable material can be positioned between one or more high voltage electrodes and the nail surface. This highly polarizable material can be polarizable fluid like water, gel, thin film of electrically conducting paint or epoxy, or even ionized gas (plasma). In some embodiments, the polarizable material will cover the surface of the nail in a conformal fashion in order to create the electric field within the nail and the nail bed uniformly under the surface of the nail. In other embodiments, the polarizable material may cover a portion of the entire nail. The polarizable material may also cover one nail or multiple nails simultaneously.

In the embodiments where plasma is employed as the highly polarizable material covering the entire nail surface or its portion, this plasma can be formed proximate to the outer surface of the nail in a variety of ways. For example, this plasma can include a corona discharge plasma, a dielectric barrier discharge plasma, plasma "jets" of various kinds where generation of plasma on the outer surface of the nail is assisted by a flow of gas toward the nail, an inductively coupled plasma, a microwave induced plasma and/or capacitively coupled radio frequency induced plasma. Tissue under the nail can be effectively connected through the body to a second electrode during the plasma generation on the surface of the nail or it may by electrically disconnected from any electrodes and remain at a floating potential whose value is determined by a variety of factors including tissue properties, body size, quality of plasma above the surface of the nail and others.

Creating a strong electric field within nail bed and within the nail may be accomplished via pulsation of the voltage applied to the polarizing fluid covering the outer surface of the nail. This is due to the fact that the nail bed tissue and possibly the nail contain a certain amount of electrolyte having electrical conductivity on the order of 1 S/m. It is known that any electrically conducting medium including electrolytes found in tissues can sustain electric field for only a limited amount of time before sufficient separation of electrical charges in the tissue electrolytes screens the electric field from within the tissue bulk bringing the net field magnitude there to zero. One can roughly estimate using the following expression for the electric field in linear conducting materials as a function of time:

$$\frac{\partial E(r,t)}{\partial t} + \frac{\sigma}{\varepsilon} E(r,t) = 0$$

where $E(r,t)$ is the electrostatic field at locations in space indicated by vector $r$ from the origin of the coordinate system at a time t, while $\sigma$ is the tissue conductivity and $\varepsilon$ is its dielectric constant. This expression follows directly from the fact that the sum of the displacement and conduction currents in tissue is zero after the initial field excitation by a voltage pulse applied to the surface of the nail. The above equation can be easily solved to yield:

$$E(r,t) = E_0(r)\exp\left(-\frac{\sigma}{\varepsilon}t\right) = E_0(r)\exp\left(-\frac{t}{\tau}\right)$$

where $E_0(r)$ is the initial value of the electrostatic field at any position $r$ and $\tau = \dfrac{\varepsilon}{\sigma}$ is the characteristic time during which the screening occurs and the initial electrostatic field effectively decays to zero in tissue. Assuming that the permittivity of tissue is about the same as that of water, $\varepsilon \approx 10^{-9}$ Farads per meter. Taking the tissue conductivity to be about $\sigma = 1$ Siemens per meter we find the characteristic time to be about $\tau = 1$ nanosecond. From these very simple estimates it can be concluded that the electric field can penetrate conducting tissue for a period of few nanoseconds. The actual electric field penetration time can vary from 1 to 1000 ns depending on the actual conductivity of the nail and nail bed that is likely to be below 1 Siemens per meter. Thus, to achieve longer effective time of the electrostatic field presence in the nail and nail bed, it is important to repeat voltage pulses whose duration can be varied from about 1 ns to 1000 ns, several times per second. Frequency of such pulses may also be varied from only a few Hertz to several thousand Hertz.

In accordance with further aspects, the techniques disclosed herein can be used in combination with the application of heating (via conduction, infrared light, plasma, or other electrical) or cooling. In accordance with a preferred embodiment, heating is also applied to the tissue being treated. Thus plasma can be applied in addition to the heating, such that the tissue is being exposed to heat, reactive ion species generated by the plasma, light emission from the plasma, and electric field generated within the plasma. The heat can be generated in whole or in part by the plasma or in combination with a second heating source. By way of further example, most or all of the heat can be provided from a source in addition to the plasma. Such a source of heat can included a resistive heater, convective heater (forced air), infrared LED's, heating lamps, and the like.

Additional features are disclosed herein to facilitate the safe usage of exemplary devices by untrained personnel to treat differently-shaped portions of the body. These include safety protections, control schemes, ergonomic holding structures, electrode structures, and spacing means, among other features. Estimated treatment time is preferably at least a tenth of a second and preferably no more than 1 hour, and in any desired time increment therebetween in increments of one minute or a multiple of minutes or in increments of one second or multiple seconds, as desired.

Thus, in accordance with one embodiment, a system for applying a plasma discharge is provided. The system includes an electrode adapted to be placed proximate an anatomical region of interest, a power supply in electrical communication with the electrode, the power supply being adapted and configured to apply power to the electrode to generate a plasma proximate the electrode.

In some implementations, the electrode can be flexible and/or resilient. The electrode can be shaped like a stylus having an insulating tip (e.g., of silicone) to permit plasma to form along the surface of the tip, and along the surface of the nail being treated. The power supply can be adapted and configured to apply a pulsed voltage waveform to the electrode to generate a plasma proximate the electrode. The pulsed voltage waveform can have pulses with durations that are shorter or longer than the time required for the formation of microdischarges between the electrode and the anatomical region of interest. If desired, the electrode can be substantially inflexible. The pulse duration of the waveform can be between at least one of (i) about 0.000000010 seconds and about 0.00000010 seconds, (ii) about 0.0000000010 seconds and about 0.000000010 seconds, (iii) about 0.00000000010 seconds and about 0.0000000010 seconds, (iv) about 0.000000001 seconds and about 0.001 seconds, and (v) about 0.000001 seconds and about 0.001 seconds. The flexible electrode can include a layer of conductive material. If desired, the system can further include a flexible dielectric layer substantially surrounding the flexible electrode, the flexible dielectric layer being adapted and configured to be disposed against the anatomical region of interest. The layer of conductive material can be a continuous layer or an interrupted layer. The interrupted layer can be etched and/or a mesh, or be in a predetermined pattern. In some embodiments, at least a portion of the layer of conductive material can be transparent, and if desired, include indium tin oxide (ITO).

If desired, the layer of conductive material can be a conductive fluid disposed within the dielectric layer. The dielectric layer and conductive fluid can be formed into a shape matching the tissue (e.g., nail plate). If desired, the dielectric layer can include an adhesive layer for placement against the region of interest to hold the flexible electrode against the tissue (e.g., nail). The system can further include a removable protective layer disposed on the adhesive layer.

In an alternative embodiment, the electrode can have a single curved dielectric layer or a plurality of curved bumps such that the curvature(s) is (are) opposite the curvature of the nail plate (i.e. defining small area(s) or point(s) of contact between the nail plate and dielectric-covered electrode. With this configuration, the electric field (and resulting plasma in some instances) will be more readily concentrated at and below the point(s) of contact within the nail plate and any pores that are present. Simultaneously, some plasma will usually form around the periphery of the curved dielectric layer(s) in use and conduct to the surface of the nail plate. This plasma is helpful for killing any fungus, bacteria, or other infectious microorganisms that may be present on the surface of the nail. In this fashion, the infection may be killed within the nail plate and on top of the nail plate simultaneously. By moving such an electrode over the entire surface of the nail plate and surrounding nail matrix, it is possible to treat the entire nail. Such methods include serpentine, spiral, or random path scanning or step-and-repeat stationary exposures in an array across the entire area such as by using an electrode having an array of conductors that can be selectively activated and deactivated to help establish desired treatment goals. A conducting pad can be placed near the anatomical region of interest (e.g., under the toe(s) of the patient) and the current passing through the conducting pad can be measured constantly by a controller of the system that adjusts the amount of applied voltage to establish a desired electric field and/or magnitude of plasma formation in the treatment area.

An additional benefit from using a curved electrode or array of curved bumps on the electrode is that the distance between the dielectric layer and the nail plate will necessarily vary. It is possible to maintain a variable distance also with a flat electrode, so long as the nail plate is sufficiently curved. This variable distance generally makes it more difficult for plasma to form microdischarges (or "sparks") that self-organize and create strong local current flows. Such strong local current flows can lead to excessive local heat generation and pain.

In some embodiments, the system can further include a gas supply in operable communication with the electrode, wherein the gas supply can be adapted and configured to supply gas to the anatomical region of interest. If desired, the system can further include a fastener for holding the flexible region against the anatomical region of interest. The fastener can include at least one of (i) a hook and loop fastener, (ii) adhesive and (iii) an elastic strap, as desired.

The system can further include an exposure indicator. The exposure indicator can be adapted to indicate the amount of exposure of the anatomical region of interest to the plasma. In some embodiments, the exposure indicator can include at least one compound that reacts to the exposure from plasma. The exposure indicator can provide a visual indication of exposure to plasma. The exposure indicator can change color when exposed to plasma. The exposure indicator can include an optical sensor in operable communication with a processor adapted and configured to control the power supply. The exposure indicator can include an electrical sensor in operable communication with a processor adapted and configured to control the power supply.

In some embodiments, the system can further include a controller for controlling the power supply. The controller can be adapted and configured to receive operational data indicative of the operation of the system, to process the operational data, to determine at least one action to take in response to the processed data, and to implement the at least one action. The electrode can include a continuity sensor to determine if the electrode is in adequate physical contact with the anatomical region of interest. The continuity sensor can be adapted and configured to measure the impedance of tissue which the sensor is in contact with. The operational data can relate to at least one of (i) a tissue impedance measurement, (ii) gas temperature, (iii) tissue temperature, (iv) light emission of the plasma, and (v) electrical current flowing into the tissue. The conditions sustaining the plasma can be modulated in response to the operational data. The conditions that are modulated can include at least one of (i) a change in the pulse shape of a waveform applied to the electrode, (ii) the frequency of the applied waveform, (iii) the voltage of the applied waveform and (iv) flowrate of a gas used to help sustain the plasma.

In further embodiments, the system can further include a ground pad for providing a ground to prevent injury to tissue in the anatomical region of interest. The ground pad can be integrated into the electrode. The ground pad can be embedded into the electrode, or can be formed about a periphery of the electrode. The ground pad can alternatively be separate from the electrode. The flexible electrode can be adapted to be applied to the anatomical region of interest without an intervening dielectric layer.

The disclosure further provides a system for applying a plasma discharge. The system includes a flexible electrode adapted to be placed proximate an anatomical region of interest, and a power supply in electrical communication with the flexible electrode, the power supply being adapted and configured to deliver power to the electrode to generate a plasma between the electrode and the anatomical region of interest. The plasma can be a corona discharge plasma, a dielectric barrier discharge plasma, a microdischarge plasma, an inductively coupled plasma, a microwave induced plasma, or a capacitively coupled radio frequency induced plasma.

In some embodiments, the flexible electrode can include a layer of conductive material. The system can further include a flexible dielectric layer substantially surrounding the flexible electrode, the dielectric layer being adapted and configured to be disposed against the anatomical region of interest. The layer of conductive material can be a continuous layer or an interrupted layer. The interrupted layer can be etched, and/or be a mesh. The layer of conductive material may be, transparent, and may include indium tin oxide (ITO), as desired.

The disclosure further provides a method of generating a plasma discharge. The method includes providing an electrode adapted to be placed proximate an anatomical region of interest, and applying a pulsed voltage waveform to the electrode to generate a plasma proximate the electrode, the pulsed voltage waveform having pulses with durations that are shorter than the time required for the formation of microdischarges between the electrode and the anatomical region of interest. The waveform can have a pulse duration between at least one of (i) about 0.000000010 seconds and about 0.00000010 seconds, (ii) about 0.0000000010 seconds and about 0.000000010 seconds, and (iii) about 0.00000000010 seconds and about 0.0000000010 seconds. The electrode can be flexible.

The power deposited by the plasma on the anatomical region of interest can be between about 1.0 milliwatts per square centimeter and about 10.0 watts per square centimeter. The power deposited by the plasma on the anatomical region of interest can be between about 10.0 milliwatts per square centimeter and about 1.0 watts per square centimeter. The power deposited by the plasma on the anatomical region of interest can be between about 100.0 milliwatts per square centimeter and about 0.5 watts per square centimeter. The anatomical region of interest can be exposed to the plasma for between about one tenth of a second and about one hour. The anatomical region of interest can be exposed to the plasma for between about five seconds and about fifteen minutes. The anatomical region of interest can be exposed to the plasma for between about thirty seconds and about ten minutes. The anatomical region of interest can be exposed to the plasma for between about three minutes and about seven minutes.

The disclosure provides a method of treating a disorder in a treatment area. The method includes generating a plasma proximate the treatment area, and causing reactive ion species in the plasma to interact with tissue in the treatment area including the disorder, such as an infectious disorder, such as onychomycosis and/or some other nail disease such as psoriasis or other infection. The disorder can be on or in an animal or human. The plasma can be a corona discharge plasma, a dielectric barrier discharge plasma, a microdischarge plasma, an inductively coupled plasma, a microwave induced plasma, a plasma jet, or a capacitively coupled radio frequency induced plasma.

In further aspects, the method can further include controllably flowing a gas proximate the treatment area. The gas composition and flowrate can be selected to accomplish at least one of (i) exposing the treatment area to a desired wavelength spectrum of light, (ii) heating the treatment area, (iii) directing electrical current through the treatment area and (iv) delivering chemical species to the treatment area. Reactive oxygen chemical species are delivered to the treatment area in accordance with any embodiment herein. The wavelength spectrum and the intensity of the light can be selected to stimulate blood flow to the treatment area. At least some of the light can be in (i) the near-infrared range, (ii) the infrared range, (iii) the ultraviolet range and (iv) the visible range. At least some of the light can be in the UVA range, and the method can further include applying psoralen to the treatment area. Reactive nitrogen species can be present in the plasma. At least some of the light can be in the UVB range, and the tissue (e.g., nail) disorder can be psoriasis or vitiligo. The methods can further include applying a sensitizing material to the treatment area prior to application of the plasma to the treatment area. Similarly, the methods can include applying a blocking material to tissue proximate the treatment area to protect the tissue from plasma.

In accordance with further aspects, the power deposited by the plasma on tissue in the treatment area including the disorder can be between about 10.0 milliwatts per square centimeter and about 1.0 watts per square centimeter. The power deposited by the plasma on the tissue in the treatment area including the disorder can be between about 100.0 milliwatts per square centimeter and about 0.5 watts per square centimeter. The tissue in the treatment area including the disorder can be exposed to the plasma for between about thirty seconds and about ten minutes in any desired time increment of one second. For example, the tissue in the treatment area including the disorder can be exposed to the plasma for between about three minutes and about seven minutes.

The disclosure further provides a method of treating an infection (e.g., a fungal based infection) in a treatment area. The method includes generating a plasma proximate the treatment area, and causing reactive ion species in the plasma to interact with infected tissue in the treatment area. The infection can be on or in an animal or human. The infection can be a bacterial, fungal, viral, or parasitic infection. The plasma can be a corona discharge plasma, a dielectric barrier discharge plasma, a microdischarge plasma, an inductively coupled plasma, a microwave induced plasma, a plasma jet, or a capacitively coupled radio frequency induced plasma. The method can further include controllably flowing a gas proximate the treatment area. The gas composition and flowrate can be selected to accomplish at least one of (i) exposing the treatment area to a desired wavelength spectrum of light, (ii) heating the treatment area, (iii) directing electrical current through the treatment area, (iv) delivering chemical species to the treatment area. Reactive oxygen chemical species can be delivered to the treatment area. The wavelength spectrum and the intensity of the light can be selected to stimulate blood flow to the treatment area. At least some of the light can be in (i) the near-infrared range, (ii) the infrared range, (iii) the ultraviolet range and (iv) the visible range. The method can further include applying a sensitizing material to the treatment area prior to application of the plasma to the treatment area, and or applying a blocking material to tissue proximate the treatment area to protect the tissue from plasma.

In some further aspects, the power deposited by the plasma on tissue in the treatment area including the disorder can be between about 10.0 milliwatts per square centimeter and about 1.0 watts per square centimeter. The power deposited by the plasma on the tissue in the treatment area including the disorder can be between about 100.0 milliwatts per square centimeter and about 0.5 watts per square centimeter. The tissue in the treatment area including the disorder can is exposed to the plasma for between about thirty seconds and about ten minutes. The tissue in the treatment area including the disorder can be exposed to the plasma for between about three minutes and about seven minutes

In accordance with further aspects, the method can further include providing an exposure indicator, the exposure indicator being adapted to indicate the amount of exposure of the tissue to be treated to the plasma, and detecting the exposure of the tissue to the plasma. The exposure indicator can include at least one compound that reacts to the exposure from plasma. The exposure indicator can provide a visual indication of exposure to plasma. The method can further include applying a sensitizing material to the tissue to be treated prior to application of the plasma and/or applying a blocking material to tissue proximate the treatment area to protect the tissue proximate the treatment area from plasma.

The disclosure further provides a processor-readable computer program stored on a tangible non-transient medium for operating a plasma treatment device including a controller, a power source operably coupled and controlled by the controller, and an electrode in operable communication with the power source and controller. The program includes instructions to cause the controller to operate the power source to induce a plasma between the electrode and a treatment area, or any other method step or aspect of any system recited in this disclosure. For example, the plasma treatment device can further include a controllable gas delivery system for directing gas to the treatment area, and the computer program can further include instructions for controlling the flow of gas to the treatment area.

In accordance with further aspects of the disclosure, a method of treating an infection, such as a fungal infection, is provided that includes providing an electrode adapted to be placed proximate an anatomical region of interest having a fungal infection, and applying an electric field to the region of interest to kill the fungal infection. The method can alternatively include providing an electrode adapted to be placed proximate an anatomical region of interest having a fungal infection, and applying a plasma to the region of interest to kill the fungal infection.

In accordance with further aspects, the fungal infection can include onychomycosis, psoriasis and the like. The method can include applying a pulsed voltage waveform to the electrode to generate a plasma proximate the electrode, the pulsed voltage waveform having pulses with durations that are shorter than the time required for the formation of microdischarges between the electrode and the anatomical region of interest. For example, the waveform ca have a pulse duration between at least one of (i) about 0.000000010 seconds and about 0.00000010 seconds, (ii) about 0.0000000010 seconds and about 0.000000010 seconds, and (iii) about 0.00000000010 seconds and about 0.0000000010 seconds. The electrode can be flexible and/or resilient. The power deposited by the plasma on the anatomical region of interest can be between about 1.0 milliwatts per square centimeter and about 10.0 watts per square centimeter, between about 10.0 milliwatts per square centimeter and about 1.0 watts per square centimeter, or between about 100.0 milliwatts per square centimeter and about 0.5 watts per square centimeter, among others. The anatomical region of interest can be exposed to the plasma for between about one tenth of a second and about one hour, between about five seconds and about fifteen minutes, between about thirty seconds and about ten minutes, and between about three minutes and about seven minutes, among others.

In accordance with still further aspects, the anatomical region of interest can be wetted with a beneficial agent during the treatment or not wetted with a beneficial agent. As such, the anatomical region of interest can be wetted with a beneficial agent prior to applying plasma or an electric field to the region of interest. The beneficial agent can be water, and/or one or more materials selected from the group including organic materials, gaseous materials, gelatinous materials, liquid materials amino acids, saline, deionized water, and phosphate buffered saline. If desired, a plasma and/or an electric field can be applied to the region of interest before being wetted and after being wetted. The plasma and/or electric field can be applied to the region of interest more than once when the region of interest is wetted. The infection being treated can be in and/or on an animal or a human or other location where the infection is present. For example, a fungal or other infection could be present on a plant, building surface or other object to be disinfected.

In accordance with further aspects, the plasma can be a corona discharge plasma, a dielectric barrier discharge plasma, a microdischarge plasma, an inductively coupled plasma, a microwave induced plasma, a plasma jet, or a capacitively coupled radio frequency induced plasma. The method can further include controllably flowing a gas proximate the region of interest. The gas composition and flowrate can be selected to accomplish at least one of (i) exposing the region of interest to a desired wavelength spectrum of light, (ii) heating the region of interest, (iii) directing electrical current through the region of interest, (iv) delivering chemical species to the region of interest. In any embodiment herein, reactive ion chemical species can be delivered to a desired location, or region of interest, and/or such reactive ion chemical species can be created in situ, such as by application of an electric field, although the presence of an electric field on its own independent of the presence of plasma formation is believed by Applicant to have therapeutic benefit. The wavelength spectrum and the intensity of the light can be selected to stimulate blood flow to the region of interest. At least some of the light can be in (i) the near-infrared range, (ii) the infrared range, (iii) the ultraviolet range and/or (iv) the visible range. Some of the light can be in the UVA range, and the method can further include applying psoralen to the region of interest. In some implementations, reactive oxygen species and/or reactive nitrogen species can be delivered to the region of interest. In other implementations, at least some of the light can be in the UVB range, and the infection can include psoriasis or vitiligo. If desired, the method can further include applying a sensitizing material to the region of interest prior to application of plasma to the region of interest. A blocking material can be applied to tissue proximate the region of interest to protect the tissue from plasma. Tissue in the region of interest including the disorder can be exposed to the plasma for between about thirty seconds and about ten minutes. Tissue in the region of interest including the disorder can be exposed to the plasma for between about three minutes and about seven minutes.

The disclosure still further provides a processor-readable computer program stored on a tangible non-transient medium for operating a plasma treatment device or device adapted to administer an electric field to a region of interest, including a controller, a power source operably coupled and controlled by the controller, and an electrode in operable communication with the power source and controller, wherein the program includes instructions to cause the controller to operate the power source to induce a plasma and/or apply an electric field between the electrode and the region of interest. The plasma treatment device can further include a controllable gas delivery system for directing gas to the region of interest, and the computer program can further include instructions for controlling the flow of gas to the region of interest.

The disclosure further provides a system for applying a plasma discharge and/or electric field. The system includes a controller, an electrode in operable communication with the controller, the electrode being adapted to be placed proximate an anatomical region of interest, the electrode having an array of conductors adapted and configured to be selectively activated and deactivated by the controller. The system further includes a power supply in electrical communication with the electrode and controller, the power supply being adapted and configured to apply power to the electrode to generate a moving plasma over time in the anatomical region of interest by activating a plurality of conductors in the array of conductors.

In accordance with further aspects, the electrode can be made from material that is resilient and/or flexible. The power supply can be adapted and configured to apply a pulsed voltage waveform to the electrode to generate a plasma proximate the electrode. The pulsed voltage waveform can have pulses with durations that are shorter than the time required for the formation of microdischarges between the electrode and the anatomical region of interest, if desired. If desired, the electrode can be substantially inflexible. The waveform can have a pulse duration between at least one of (i) about 0.000000010 seconds and about 0.00000010 seconds, (ii) about 0.0000000010 seconds and about 0.000000010 seconds, (iii) about 0.00000000010 seconds and about 0.0000000010 seconds, (iv) about 0.000000001 seconds and about 0.001 seconds, and (v) about 0.000001 seconds and about 0.001 seconds. The system can further include a conducting pad adapted to be disposed at a location remote from the electrode in electrical communication with the electrode through the anatomical region of interest. The controller can be adapted and configured to adjust the applied voltage in response to a signal received from the conducting pad to maintain a controlled and substantially uniform power deposition in the anatomical region of interest. The electrode can have a distal tip made from a resilient material, wherein the distal tip can have a rounded distal tip to facilitate formation of a surface plasma thereon.

In further accordance with the disclosure, a kit is provided including a beneficial agent as described herein in a dispenser and a treatment tip for a plasma treatment device. The dispenser can be adapted and configured to dispense beneficial agent onto a nail plate. The beneficial agent can include a conductive gel for application to a nail plate. The beneficial agent can include a material selected from the group consisting of organic materials, gaseous materials, gelatinous materials, liquid materials amino acids, saline, deionized water, and phosphate buffered saline.

The disclosure further provides a method of treatment, including providing an electrode adapted to be placed proximate an anatomical region of interest to be treated, and applying an electric field to the region of interest to treat the anatomical region of interest. The treatment can be performed to treat an infection. The infection can include a fungal or other infection. Illustrative fungal infections in clued onychomycosis and psoriasis. The method can further include applying a pulsed voltage waveform to the electrode to generate a time varying electric field over the anatomical region of interest, the pulsed voltage waveform having pulses with durations that are shorter than the time required for electrical charges in electrolytes in the tissue to screen the electric field. The pulsed waveform can have a pulse duration between at least one of (i) about 0.000000010 seconds and about 0.00000010 seconds, (ii) about 0.0000000010 seconds and about 0.000000010 seconds, and (iii) about 0.00000000010 seconds and about 0.0000000010 seconds. The electrode can be flexible and/or resilient, or may not be flexible or resilient. The power deposited by the electric field in the anatomical region of interest can be between about 1.0 milliwatts per square centimeter and about 10.0 watts per square centimeter, between about 10.0 milliwatts per square centimeter and about 1.0 watts per square centimeter, or between about 100.0 milliwatts per square centimeter and about 0.5 watts per square centimeter, among others. The anatomical region of interest can be exposed to the electric field for between about one tenth of a second and about one hour. The anatomical region of interest can be exposed to the electric field for between about five seconds and about fifteen minutes, between about thirty seconds and about ten minutes, or between about three minutes and about seven minutes, among others. The anatomical region of interest may or may not be wetted with a beneficial agent during the treatment. The anatomical region of interest can be wetted with a beneficial agent prior to applying the electric field. The beneficial agent can be water, and/or organic materials, gaseous materials, gelatinous materials, liquid materials amino acids, saline, deionized water, and phosphate buffered saline. The electric field can be applied before being wetted and/or after being wetted. The electric field can be applied more than once when the region of interest is wetted. The anatomical region of interest can be on or in an animal, a human, or other living tissue or inanimate object to be disinfected. The electric field can have a strength between about 3,oooV/mm and 20,000 V/mm, among others. In some implementations, the electric field may not result in substantial formation of plasma in the anatomical region of interest. In some instances, the electric field may result in no detectable plasma formation in or on the anatomical region of interest.

It is to be understood that both the foregoing general description and the following detailed description are exemplary and are intended to provide further explanation of the embodiments disclosed herein. The accompanying drawings, which are incorporated in and constitute part of this specification, are included to illustrate and provide a further understanding of the methods and systems of the disclosure. Together with the description, the drawings serve to explain the principles of the disclosed embodiments.

BRIEF DESCRIPTION OF THE DRAWINGS

Fig. 1 is a schematic showing an exemplary single, rigid plasma electrode, high voltage supply and treatment of a nail.
Fig. 2 is a schematic showing an exemplary multi-electrode device and how it may be used to treat multiple nails.
Fig. 3 is a schematic of an exemplary semi-flexible to flexible plasma electrode.
Fig. 4 is a schematic of an exemplary plasma-jet device and its application to a nail.
Fig. 5 is a schematic showing an exemplary treatment electrode employing a surface plasma.
Fig. 6 is a schematic showing an exemplary treatment electrode employing small holes to help initiate the plasma formation at lower voltages and/or less complex waveforms.

Fig. 7 is a schematic showing an exemplary flexible treatment electrode with an integrated spacer.

Figs. 8a-8b illustrate side and top views of an assembly for testing formation of plasma in small gaps.

Figs. 9a-9c present photographic evidence of plasma discharge formation.

Fig. 10 is a depiction of an exemplary plasma treatment device for treating onychomycosis and other infections.

DETAILED DESCRIPTION

Reference will now be made in detail to the present preferred embodiments of the disclosure, examples of which are illustrated in the accompanying drawings. The methods and corresponding steps of the disclosed embodiments will be described in conjunction with the detailed description of the exemplary systems.

By way of introduction, plasma, sometimes referred to as the "fourth state of matter", typically includes partially and/or fully ionized gas molecules and can be produced and directed in a variety of ways and geometries. More specifically, a plasma can be thought of as a gas having molecules that can be partially or fully ionized and electrons that have kinetic energy sufficient to strip at least one electron from at least one of the gas molecules through collisions, such that the resulting plasma includes a mixture of positively charged ions in a sea of free electrons that may or may not also include neutral species mixed therewith. Plasmas can be used for a variety of purposes, including sterilization, blood coagulation, ozone generation, chemical processing, light sources, ion sources (for propulsion) and heat sources, among others. As a result of the relative simplicity of the construction of gas discharges as opposed to other emitters, such as solid state lasers, it is possible to create a variety of structures to provide a distributed energy source at an economical cost. Perhaps the best example of such arrays is the plasma television.

Plasma is most often generated in some region of gas when the electric field in this region exceeds a certain breakdown value. This breakdown value may depend on a number of factors including gas pressure, the type of gas, and the size of the region. In atmospheric pressure air, when the size of the plasma generation region exceeds several times the mean free path, the break down field is about 3,000 Volts per millimeter. In practice, the size of the region where this field needs to be exceeded to generate plasma is on the order of few micrometers. For smaller regions, it may not be possible to generate plasma within them unless the electric field is substantially larger. Thus, as long as the pores within the nail and the nail bed are larger than few micrometers (1-2 micrometers), it should be possible to generate plasma within these pores using electric fields in the range of about 3,000-20,000 Volts per millimeter in increments, for example, of one volt/mm (e.g., 3001 V/mm, 3002V/mm...). However, larger (e.g., 20,000-50,000 V/mm) or smaller (e.g., 100V/mm-3,000V/mm) electric fields can be used, as desired, as the present disclosure can provide merely applying electric field irrespective of whether plasma is created as a result of the applied electric field. Since the electric field in the nail and the nail bed can be generated remotely, without necessarily direct contact with conducting electrode, plasma in the pores of the nail and the nail bed can also be generated remotely from any current conducting electrode.

In accordance with the present disclosure, onychomycosis treatment methods and related systems and machine readable programs have been developed using atmospheric pressure plasmas and/or electric fields, alone or in combination with other therapies described herein. Atmospheric plasmas are typically considered to be those plasmas that can exist in a room environment at standard conditions or conditions that vary slightly therefrom (e.g. at standard temperature and pressure "STP"). The plasma can be a corona, dielectric barrier discharge, microdischarge; inductively coupled plasma, microwave induced plasma, or capacitively coupled radio frequency induced plasma. The plasma can also be induced as the result of a laser exposure. In one embodiment, plasma is created within the nail for a duration of at least one tenth of a second and no more than one hour, or any duration therebetween in increments of minutes, seconds, or tenths of seconds, as desired. Other embodiments are also presented.

The plasma produces reactive chemical species such as hydroxyl radicals (OH), nitrous oxide ($NO_2$), nitric oxide (NO), ozone ($O_3$), superoxide ($O_2^-$) that kill the pathogens responsible for the onychomycosis. The plasma can also emit light of a variety of wavelengths, can generate heat, ions, and electrons. The combination of these species and energy emissions can react with or cause reactions within the tissue that can affect the local cellular makeup, inflammation or other cellular processes and thereby alleviate the symptoms of various conditions.

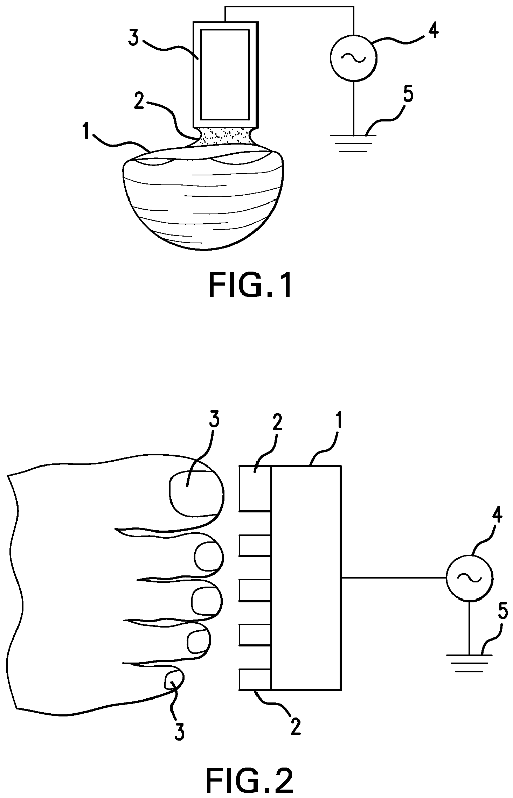

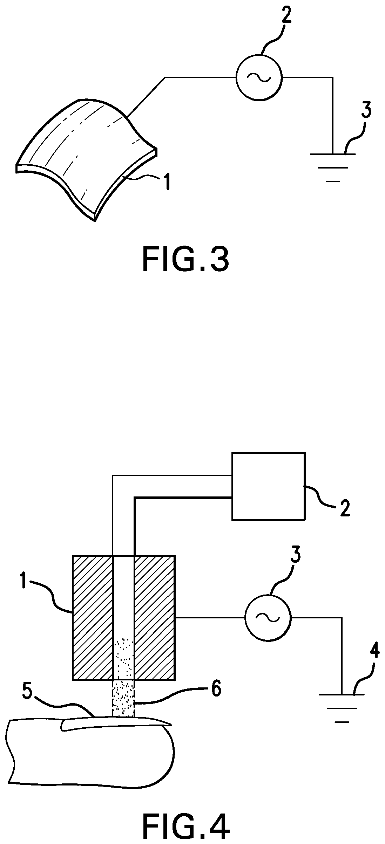

For purposes of illustration only, and not limitation, Figure 1 shows a schematic of an exemplary treatment electrode and nail treatment method in accordance with the disclosure. A nail (fingernail or toenail), 1, has a conductive media, 2, placed upon it. The treatment electrode, 3, which can include a conductor that is encapsulated by a dielectric layer, is placed in contact with the conductive media, 2. The treatment electrode is connected to a high-voltage power supply and control system, 4, which is connected to an electrical ground, 5. Figure 2 shows a schematic of an electrode structure that can treat multiple nails at one time. The electrode support structure, 1, has multiple treatment heads, 2, which are placed and sized appropriately to address individual nails, 3. The electrode support structure, 1, is connected to a high voltage power supply and control system, 4, which is connected to ground, 5. Figure 3 shows a schematic of a flexible or semi-flexible treatment electrode. This flexible or semi-flexible treatment electrode, 1, can be bent to conform to the shape of the target nail structure (not shown). The treatment electrode, 1, is connected to a high voltage power supply and control system , 2, which is connected to an electrical ground, 3. Figure 4 shows a schematic of a plasma jet device. The treatment electrode, 1, is connected to a gas supply, 2, which provides a stream of gaseous media that is excited into a plasma state when the high voltage power supply and control system, 3, is activated. The high voltage power supply and control system, 3, is connected to an electrical ground, 4. Note that in this case, no external conductive media is needed to be applied to the nail, 5, because the gas supply (which becomes a plasma, 6) provides this conductive function.

In accordance with the disclosed embodiments, the treatment electrode may include multiple materials and have multiple shapes and surface finishes. Some example materials include aluminum or other conductor and alumina ($Al_2O_3$) dielectric, copper or other conductor and silicon nitride dielectric, conductor and quartz dielectric, conductor with rubber or plastic dielectrics (such as a metal conductor with silicone or epoxy with or without glass reinforcement), and conductor with a foam dielectric (such as silicone, polyurethane, or polyethylene foam). The choice of the dielectric material is based on the dielectric breakdown strength, dielectric constant, and the intended duration of usage. Some material combinations may be more suitable for long-term usage (such as copper and quartz), whereas other material combinations may be more suitable for short-term or single time usage. In the case of a foam dielectric, the pores of the foam are designed such that a microdischarge may form in each of a plurality of pores. These microdischarges are sufficiently numerous such that no individual microdischarge has sufficient energy to cause damage, pain, erythema, or irritation. The dielectric layers have a minimum thickness of about 10 microns and are attached to the conductor, for example, by molding, laminating, bonding, brazing, welding, mechanical joining. Alternatively, the dielectric layer may be applied via a coating process, such as anodizing or thermal spraying or by an oxidation process. The shape of the conductor may be flat or curved, which will affect the distribution, location and intensity of the plasma created. If the treatment electrode is smaller than the affected tissue area, then the operator will have to sweep the electrode over the desired treatment area to generate the plasma where required. Alternately, the treatment electrode may have the same size or substantially the same size as the desired treatment area, in which case the operator can apply the electrode in contact with the desired treatment area and maintain its position for the duration of treatment. The connection of the treatment electrodes to the electrical support structure may be rigid or adjustable.

In order to prevent formation of powerful microdischarges that bridge the gap between the electrode surface and tissue (e.g., nail) and remain in one specific location on the tissue (e.g., nail) for a period longer than about 1 second, one or more of the following exemplary techniques can be used:

Electrodes having non-uniform air (gas) gap and some portions of the electrode surface extending so as to be in or near contact with tissue (e.g., nail) can be used to create plasma on the electrode surface and guide this surface plasma toward the tissue (e.g., nail) localizing around the point of contact or near contact between the electrode and the tissue (e.g., nail).

Scanning the electrode rapidly (manually or with a motor) across the tissue (e.g., nail) so as to treat areas that may not be sufficiently exposed to the plasma when the electrode is immobile.

Use of high voltage waveforms that are similar to pulses having rise time and fall time in the range between 1 picosecond and 100 nanoseconds so as to form plasma where strong microdischarges do not have sufficient time to be created.

Varying the electrode Z-position (that is, the gap between the electrode and the tissue (e.g., nail)) via vibration, oscillation or other motions (such as with a piezomotor or other oscillatory motor) such that plasma is formed between different portions of the electrode area and the tissue (e.g., nail), depending on the magnitude of the gap.

Use of microdischarge electrodes having sub-millimeter sizes and applying them in stationary or scanning exposures.

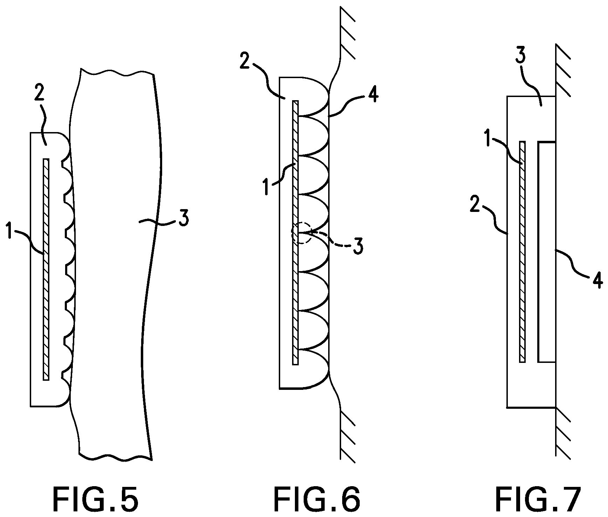

As shown in Figure 6, small openings or holes can be defined in the dielectric layer. These holes can change the nature of the plasma discharge. The characteristic dimension of the microdischarges is on the order of 100 to 200 microns (diameter). As shown in Figure 5, when the hole diameter is significantly smaller than the microdischarge diameter, the amount of current that can be passed through the hole to the electrode can be significantly restricted permitting generation of non-thermal plasma possibly even without AC voltage waveform typical of a dielectric barrier discharge.

In the case of pulsed operation, devices and associated methods are provided that provide pulsed voltages over time with very short duration. In accordance with one embodiment, the pulse duration can use any suitable voltage and be between about 0.010 seconds and about 0.10 seconds. In accordance with another embodiment, the pulse duration is between about 0.0010 seconds and about 0.010 seconds. In accordance with still another embodiment, the pulse duration is between about 0.00010 seconds and about 0.0010 seconds. In accordance with yet another embodiment, the pulse duration is between about 0.000010 seconds and about 0.00010 seconds. In accordance with another embodiment, the pulse duration is between about 0.0000010 seconds and about 0.000010 seconds. In accordance with still another embodiment, the pulse duration is between about 0.00000010 seconds and about 0.0000010 seconds. In accordance with a further embodiment, the pulse duration is between about 0.000000010 seconds and about 0.00000010 seconds. In accordance with still a further embodiment, the pulse duration is between about 0.0000000010 seconds and about 0.000000010 seconds. In accordance with yet a further embodiment, the pulse duration is between about 0.00000000010 seconds and about 0.0000000010 seconds. In accordance with another embodiment, a waveform is provided with a combination of pulses selected from the durations set forth above. Use of pulses of such short duration are believed to result in decreased streamer (microdischarge) formation on the basis that the pulse is too short for the plasma to organize itself in a manner in which it can form a streamer (microdischarge). It is also believed that use of such pulsing can result in a large amount of reactive ion species for treating the tissue (e.g., nail). Moreover, it is possible to not use a dielectric material between the electrode and tissue when using pulses of such short duration, since the power applied to the area being treated is controlled by microprocessor; although a dielectric layer can be included for safety reasons. As such, this technique of using pulses of such short duration differs from dielectric barrier discharge plasmas, which require a dielectric layer to operate. Moreover, using such short pulses also results in a more uniform plasma.

In accordance with further aspects, the disclosure provides systems and methods for generating surface plasmas and techniques for applying surface plasmas to a patient's tissue (e.g., nail).

For purposes of illustration, and not limitation, a further embodiment of a treatment device is provided in Figure 5. The treatment device includes a handle (not shown) and a treatment electrode including a conductor 1 surrounded at least in part by an insulating material 2 defining an outer surface that may be placed in direct contact with a patient's tissue (e.g., nail) 3. The treatment device is used in this embodiment by applying a voltage to the conductor 1 such that a surface plasma is generated along the surface of the insulating material and between the surface of the insulating material 2 and patient's nail in areas where they are not in direct physical contact, and a gap is defined between the nail and the insulating material. The behavior of surface plasma is affected by a variety of variables, including the type and overall shape of insulating material 2 used, as well as the characteristics of surface of the insulating material 2.

If desired, the insulating material can be rigid or flexible. If flexible, insulating material 2 can be, for example, a silicone compound, synthetic rubber, polyurethane, or polyethylene. These can be applied to the conductor via lamination or the conductor can be plated or otherwise sprayed onto the base insulating material. If rigid, insulating material can be a moldable material, such as PTFE, PVDF, PC, PP and the like, and can be molded such as by injection molding. As will be appreciated, the texturing of the surface will have a surface finish that can be a result of the molding process or other processing. Thus, in one embodiment, such as where insulating material is injection molded, a mold having a surface finish in accordance with SPI/SPE A1, A2, A3, B1, B2, B3, C1, C2, C3, D1, D2 or D3 can be used. Moreover, if desired, the mold can have a first, rougher, surface finish in one region, and a second, smoother surface finish in another region.

Regardless as to how it is formed, the resulting surface of material 2 facing and/or contacting the tissue (e.g., nail) of the patient/user can be provided with a surface having a region with a mean surface roughness Ra between about 0.01-2000 microinches, 0.1-1000 microinches, 1-100 microinches, 5-50 microinches, 20-40 microinches, 100-200 microinches, 75-125 microinches, 1-4 microinches, 4-8 microinches, 8-12 microinches, 12-20 microinches, 20-30 microinches, 30-40 microinches, 40-50 microinches, 50-60 microinches, 70-80 microinches, 80-90 microinches, 90-100 microinches, or the like.

The surface of insulating material 2 that faces and/or contacts a user's/patient's tissue (e.g., nail) can be provided with one or more bumps, ridges or undulations 78 that are distinct and on a generally larger scale than the surface finish, having an average height of about 0.01mm-5mm, 0.1-0.5 mm, 0.5-1.0mm, 1.0-1.5mm, 1.5-2.0mm, 2.0-2.5mm, 2.5-3.0mm, 3.0-3.5mm, 3.5-4.0mm, 4.0-4.5mm, or 4.5-5.0mm, among others. Distances between adjacent bumps, ridges or undulations for the foregoing examples can be between 0.01mm-5mm, 0.1-0.5 mm, 0.5-1.0mm, 1.0-1.5mm, 1.5-2.0mm, 2.0-2.5mm, 2.5-3.0mm, 3.0-3.5mm, 3.5-4.0mm, 4.0-4.5mm, or 4.5-5.0mm, among others.

The material of the dielectric can also be provided with pores. These pores can serve as microcavities for a plasma microdischarge. These pores may be connected to one another or be separate and distinct. Such pores could be regular, as in a capillary array, or irregular in distribution. The shape of the pores may be spherical, cylindrical, or other. The pores have a characteristic dimension of 0.001 to 0.100 mm, 0.100 to 0.5 mm, 0.5 to 1.0 mm, 1.0-1.5 mm, 1.5-2.0 mm, 2.0-2.5mm, 2.5-3.0mm, 3.0-3.5mm, 3.5-4.0mm, 4.0-4.5mm, or 4.5-5.0mm, among others.

If desired, insulating material can be a semiconductor material. Concentration of charge carriers (consisting of valence and conduction electrons) in semiconductors can be modulated in a variety of ways including changes in temperature, incident light and electric field inside the material. The semiconducting material properties at different locations can also be controlled through incorporation of impurities that create either excess of conduction or excess of valence electrons. Modulating charge carrier density within the semiconducting material permits to exercise control over current being delivered into the plasma. Charge carrier density within the semiconductor may also change its electron emission capabilities and the manner in which insulating material acts as an electron emitter. Furthermore, charge carrier density within the semiconducting material may result in changes of surface breakdown enabling control over surface plasma discharge on semiconductor surface.

It will be further appreciated that insulating material 2 can have a variety of different dielectric breakdown strengths, such as rubber (450 - 700 V/mil), Teflon (1500 V/mil), glass (2000 - 3000 V/mil), alumina (300 - 500 V/mil), polyimide (12000 - 18000 V/mil), PVDF (1700 V/mil), PVC, polyurethane, UHMW polyethylene, etc. By comparison, air has a dielectric breakdown strength of approximately 20 V/mil. The choice of the dielectric thickness is determined by the magnitude of the applied voltage, the gap between the dielectric and the tissue (or the profile of the dielectric, in the case of a surface discharge), the thickness of the nail plate, and the local surface profile of the tissue (which includes tissue surface roughness and topographical variations due to swelling, scarring, or gross curvature of the body). In such cases, a typical thickness of approximately 0.010 to 4 mm for the dielectric layer is suitable to account for the variations in the applied voltage, electrode-tissue separation, tissue surface profile, etc. Generally, the smaller the gap, the smaller the dielectric thickness that is required.

The minimum gap between the dielectric and the tissue (e.g., nail) can be determined according to the Paschen curve, which shows the relationship between the breakdown voltage of a gas as a function of its pressure times the characteristic distance. In some embodiments, the characteristic distance is the air gap between the dielectric and the tissue. For atmospheric pressures, the Paschen curve provides that minimum voltages of approximately 400 to 6000 volts are useful to generate a breakdown for gaps of approximately 0.01 to 1 mm, respectively. In order to form a plasma over a large area as opposed to a single microdischarge, significantly higher voltages are useful for generating plasma while overcoming the variations induced by the tissue surface roughness, tissue impedance variations, and local topographical variation of the tissue. Such voltages range, for example, from about 500 to about 1000 volts, about 1000 - about10000 volts, and about 10000 - about 50000 volts.

In further accordance with the disclosure, additional features are provided to facilitate the use of plasma treatment devices by lightly trained or untrained operators. In order to maintain the same intensity of the dose of the plasma to the tissue (e.g., nail), it is useful to apply the plasma treatment electrode in close proximity to the tissue (for cases where the curved electrode is not used) in a reliable and repeatable fashion. Alternatively, a spacer made from a non-conductive material can be used to set the distance between the plasma treatment electrode and the tissue, as shown in Figure 7, for example. The spacer/spacing means can be provided around the periphery of the treatment electrode, in which case it can also surround or encapsulate the local gas. By surrounding the local gas, the structure can facilitate concentration of the heat and reactive species in the desired treatment area. Such a border can also incorporate an ozone-absorbing material, such as carbon black, to absorb the ozone that is commonly generated by the dielectric barrier discharge. In some embodiments, a line, group of lines, a polygon or polygons, a post or a plurality of posts, such as in the form of an array, or other geometries at or around the central portion of the treatment area can be included in the electrode insulating material to prevent the tissue from rising up inside the region defined by the spacer, which would adversely affect the maintenance of a constant gap between the treatment electrode and the body. Alternately, the spacer itself can be mounted on a spring or other resilient member that provides a defined preload contact force between the plasma treatment electrode and the tissue. When combined with an overload protection interlock (such as a contact or proximity switch or sensor) to prevent operation if the spring is fully compressed, this mechanism can be used to prevent the tissue from coming too close to the plasma treatment electrode.

In another embodiment, when microdischarges are employed to generate the plasma in close proximity to the tissue to be treated (e.g., nail), the size of the microcavities is preferably small enough such that the spacing between the tissue and the plasma treatment electrodes can be controlled without additional spacing means, springs, or other mechanisms, as desired.

In accordance with some embodiments, the electrical output is delivered by a power supply and affects the nature of the plasma that is emitted. Thermal and non-thermal plasmas may be used. If desired, the power supply can be connected to a control system that provides control means (e.g., a controller) that controls turning the device on an off, and may be used to control the dose (or intensity) of the plasma, which can in turn be controlled by adjusting the gas flow rate, applied voltage and hence applied current, and the like. In order to maintain user safety, a variety of controls are preferably employed. At the point of application to the tissue, a temperature sensor (thermocouple or infrared sensor, for example) is employed to ensure that the gas temperature does not exceed the threshold for causing pain and erythema. Also, the electrode can contain a fuse or fast circuit breaker to ensure that the current does not increase dramatically as a result of electrode damage, which can cause significant pain to the patient. This fuse or circuit breaker can also be mounted within the power supply.

If desired, the controller can control a second set of conductors proximate the plasma emitters to provide a magnetic field proximate the plasma to help influence the direction of flow of the plasma as well as its density, particularly the density of free electrons within a given volume containing the tissue to be treated. Electromagnets and/or permanent magnets can be used, for example, to apply a dipole magnetic field across the tissue, thus providing magnetic field lines that are substantially oblique to the nail, thus influencing the motion of reactive species across the tissue being treated.

The electrodes that are used to generate the plasma are optionally configured to deliver the electrical energy simultaneously or sequentially. In this manner, the entire plasma emitter may be excited at one time or sequentially in lines, or sub-regions may be excited sequentially (or at the same time or substantially the same time, if desired). The control system further provides the means (by way of software or hard-wired) to excite the electrodes in the desired sequence. For sequential excitation, the electrodes or sets of electrodes are individually addressable by the control system. For sequential excitation, the control system provides the means to vary the intensity and duration of the exposure to the plasma. This variation is applied spatially, allowing the user to deliver different plasma exposure doses to different regions of the target nail or nails.

The gas delivery from a gas supply, if provided, can be controlled by a valve or set of valves. In one embodiment, the operator opens the valve to provide continuous gas flow. In an alternate embodiment, the valve or series of valves is electrically controlled via the control system.

In an alternate embodiment, there is no gas container structure. The electrodes are then used to excite the surrounding ambient air to generate the plasma. When the emitter is applied to the nail, a spacing means/spacer is used to ensure that sufficient air is available to generate the plasma that is to be directed at the nail. The spacing means can be a number of cavities, microcavities, microchannels, or other depressions having negative skewness. Alternatively, the spacing means can have positive skewness, such as posts, pillars, raised lines, or other structures that extend above the main surface of the device. The spacing means also provides isolation of the electrodes from the nail.

In order to treat the desired nail section with the plasma the following exemplary method can be used:

1. Apply a polarizable material to the target area of the nail surface. The polarizable material may be water, gel, or some other conductor.

2. Apply the plasma emitter/electrode to the polarizable material on the target area of the nail such that the emission surface(s) are aimed towards the desired treatment area. Depending on the duration of treatment, the plasma emitter may be held in place via hand pressure, gravity, or a securing means, such as an adhesive, hook and loop fastener (e.g., from Velcro, Inc.), latch, springs, or elastic straps.

3. Once the plasma emitter is in place, the user activates the device using a control means. Once activated, the emitter delivers plasma to the target nail and/or creates plasma within the target nail (as required).

4. Upon completion of the treatment, the user deactivates the device using a control means. The control means alternatively provides an automatic shutoff once the desired dose has been delivered.

5. The user then removes the plasma emitter from the target treatment area.

In an alternate embodiment, a flexible electrode is applied directly to the nail. In this case, a conductive gel is optionally applied prior to application of the flexible electrode. Such conductive gel may be integrated into an adhesive patch thereby enabling simultaneous application of the electrode and the gel.

In an alternate method to treat the target nail with plasma, sensitizing and/or blocking materials can be used to provide differential dosing for different sections of the nail. Such sensitizing materials can include water-based creams, ointments, lotions, sprays, gels, or other fluids. They can also include hydrophilic materials, such as glycerin, which can be used to attract water and water-based materials. These fluids are preferably applied topically.

The sensitizing materials can act in a variety of ways. These ways include promoting the generation of higher concentrations of specific reactive species, promoting emission of particular light wavelengths, modulating the electric field strength and direction, or other. For example, fluids such as hydrogen peroxide, saline, antioxidants such as N-acetyl cysteine (NAC) and others can react with the plasma to generate a variety of reactive species. Other suitable fluids are described, for example, in International Patent Application No. PCT/US2011/046382, filed August 3, 2011 which is incorporated by reference herein in its entirety for any purpose whatsoever. These fluids may also become highly acidic, continue to penetrate the nails post-treatment, and thereby enhance the pathogen killing efficacy. Moreover, the sensitizing material can include organic materials, as well as being in a gaseous, gelatinous or liquid state. The sensitizing material may likewise include an amino acid such as Cysteine. In certain embodiments, the sensitizing material is dissolved in a liquid. Suitable liquids include saline, deionized water, phosphate buffered saline, or a combination thereof. The amount of organic material in the sensitizing material may vary. In certain embodiments, the organic material in the is at a concentration of at least about 2.5 mM. In other embodiments, the organic material in the sensitizing material is at a concentration of at least about 5 mM. In still other embodiments, the organic material in the sensitizing material, or composition, is at a concentration of at least about 10 mM.

The blocking materials can include anhydrous (such as oil-based) creams, ointments, gels, or other fluids. They can also include hydrophobic materials which are used to repel water and water-based materials. An exemplary illustrative method can have the following steps:

1. Apply the plasma sensitizing and/or blocking materials to the desired treatment region (or protection region) of the nail. In the case of sensitizing materials, it may be necessary to wait for a certain period of time (an incubation period) for the sensitizing materials to be absorbed by the target regions of the nail.

2. Apply the plasma emitter to the target area of the nail such that the emission surface is aimed towards the desired treatment area. Depending on the duration of treatment, the plasma emitter may be held in place via hand pressure, gravity, or a securing means, such as an adhesive, Velcro, latches, springs, or elastic straps.

3. Once the plasma emitter is in place, the user activates the device using a control means. Once activated, the emitter delivers plasma to the target nail.

4. Upon completion of the treatment, the user deactivates the device using a control means. The control means alternatively provides an automatic shutoff once the desired dose has been delivered.

5. The user then removes the plasma emitter from the target treatment area.

In still another embodiment, heat, ultraviolet light, and/or infrared radiation can be applied in concert with the plasma in order to further accelerate the killing of pathogens. In another embodiment, topical and/or systemic drugs can be used in synergistic combination with the plasma treatment in order to further increase the effectiveness and speed of killing the pathogens. If thermal plasma or heat enhancements such as infrared radiation are employed, it may be desirable to cool the tissue surrounding the nail for greater patient comfort. The tissue may be cooled by flowing or spraying water or cryogen at it.

In accordance with a further aspect, a plasma sensitizer can also be used. In one aspect, the sensitizer can act as a conductive fluid to direct the plasma in a desired direction, such as toward the nail. In another aspect, the sensitizer can additionally or alternatively provide chemicals that react with the plasma, thereby enabling other reactions with the nail to occur. This can result in faster reactions at the nail. Suitable materials to be used as sensitizers can include, for example, water, saline, deionized water, or any fluid containing organic compounds, as well as materials including antioxidants.

In another embodiment, a layer containing an exposure indicator is applied to the plasma treatment electrode. By using the exposure indicator, the user will obtain direct feedback about the amount and level of exposure applied to the body. The exposure indicator can contain one or more compounds that react to the exposure from plasma such that the exposure can be detected and/or metered upon removal from the tissue. The indicator may change color or otherwise provide a visual indication of exposure. This change may occur immediately or after exposure to a developer or other chemical. An example of an exposure indicator is a photosensitive material that responds to the light emitted by the plasma. Another example of an exposure indicator is a material that changes color upon exposure to different pH levels or other chemical species, such as litmus paper. A combination of different materials may be employed to indicate different exposure levels. Such materials can be provided in sheet form, and can be replaced with each subsequent use of the treatment device if the device is otherwise intended to or capable of being reusable.

In some alternative embodiments, the exposure level is monitored automatically using optical sensors, electronic sensors, or a combination thereof. The optical sensors, for example, can detect visible, ultraviolet, or infrared emissions from the plasma. The electrical sensors can detect current flow or electrical field variation and the like as generated by the plasma emission. The information from these sensors can then be delivered to the power supply and control system to enable closed loop control of the exposure dose and intensity. Such closed loop control may be desirable to account for patient-specific anatomical or disease variations that affect the plasma intensity, for example. The gas delivery from the gas supply can be controlled by a valve or set of valves. In one embodiment, the operator opens the valve to provide continuous gas flow. In an alternate embodiment, the valve or series of valves is electrically controlled via the control system.

In another alternate embodiment, the power supply and control system are connected to the electrode by a high-voltage cable. This cable preferably has sufficient length to enable targeting any single portion of the body or multiple areas of the body. The electrode dimensions and weight are set so to enable comfortable hand gripping while a plastic or other insulating material shields the operator from any high-voltage exposure. Alternately, the electrode may be curved (i.e. to match or nearly match the curvature of the desired treatment) and/or flexible (as shown in Figure 7). A treatment electrode can have a variety of shapes, including squares, circles, rectangles, and the like that enable it to conform to the desired treatment area while maintaining the desired gap or surface discharge configuration as appropriate. The shape may be standardized for all patients or custom-made based on casting, molding, optical scanning or other measurement methods to create an electrode that more precisely conforms to the anatomy of the specific patient to be treated. Alternately, the electrode, power supply and control system can be integrated into a single handheld unit. This unit optionally contains batteries and/or a cable port to connect to a wall outlet.

In still another embodiment, heat, ultraviolet light, visible light, and/or infrared radiation can be applied in concert with or in alternating fashion with the plasma in order to further accelerate the killing of pathogens, alleviation of inflammation, and/or activation of other cellular processes and chemistry. In another embodiment, topical and/or systemic drugs can be used in synergistic combination with the plasma treatment in order to further increase the effectiveness and speed of killing and/or other reactions. In another embodiment, the electrode itself is heated and thereby provide conductive heating of the tissue, which can combine with the non-thermal plasma to enhance the effectiveness and speed of killing and/or other reactions.

If thermal plasma or heat enhancements such as infrared radiation are employed, it may be desirable to cool the tissue surrounding the treatment area for greater patient comfort. The tissue may be cooled by flowing or spraying water or cryogen at it. Alternatively, when the electrode is in contact with the tissue being treated, it can be cooled and thereby provide conductive cooling to the local tissue region being treated. In another embodiment, after-care creams, gels, or other materials may be applied to the treated tissue to help alleviate or repair pain, irritation, erythema, or other unwanted effects, such as cellular or DNA damage. For example, anti-oxidants may be used to help reduce post-treatment levels of reactive oxygen species and promote DNA repair.

In accordance with a further aspect, a plasma sensitizer can also be used. In one aspect, the sensitizer can act as a conductive fluid to direct the plasma in a desired direction, such as toward the tissue (e.g., nail). In another aspect, the sensitizer can additionally or alternatively provide chemicals that react with the plasma, thereby enabling other reactions with the tissue to occur. This can result in faster reactions at the tissue. Suitable materials to be used as sensitizers can include, for example, water, saline, deionized water, or any fluid containing organic compounds, as well as materials including antioxidants. The plasma sensitizing fluid can also be delivered to the tissue as part of the device construction. The device can contain a spray, sponge or vapor (aerosolized fluid) jet that has the sensitizing fluid and controllably releases said fluid as desired by the user or automatically upon contact by the electrode to the tissue. Finally, a moistened fabric may be placed between the electrode and the tissue. In this case, the discharge will occur within the cloth and excessive streamer formation will also be avoided.

In order to deliver higher power levels to the body, it is desirable to provide a grounding (dispersive) pad proximately located to the plasma emitter. Such pads are commonly used in conjunction with electrosurgical devices. As the current transmission increases, there is a higher risk of burning the tissue. The risk of creating tissue burns depends on the amount of current divided by the area over which it is distributed, which is also known as the current density. Nominally, the current density at the ground pad is defined by the area of the pad. However, there are some additional considerations:

1. The entire ground pad is preferably securely attached to the body of the patient. A partial attachment or removal of the ground pad can cause the current density to increase.
2. The ground pad preferably has sufficiently low resistance to avoid generation of heat within the pad. Such a resistance can range, for example, from about 0.1 to about 5000 ohms.
3. The ground pad preferably radiates any heat generated within the pad and/or can provide active cooling to minimize the risk of burning.

In order to ensure that the ground pad is attached securely to the patient, prior to treatment, remote monitoring of the pad attachment can be employed as follows. First, two or more pads or pad sections can be attached to the body in close proximity to one another. These pads can have matching connectors and a cable or cables that run back to the power supply and control system. Prior to and during treatment, the power supply and control system can send a small amount of current via one of the conductive pathways to one of the ground pads. It then measures the return current that is conducted by the second ground pad to determine the overall impedance of the system. If the measured impedance deviates from the nominal value, then the power supply and control system prevents the treatment from starting and/or interrupts the treatment. An indicator means (visual, audible, etc.) is provided on the power supply and control system to inform the operator that the grounding pad(s) are not fully attached to the body.

Optionally, the grounding pad(s) may be integrated with the plasma emitter. Such a construction may provide advantages in ease of application to the body, convenience, and/or lower cost. The grounding pad can be provided within the plasma emitter, for example, by providing a grounding conductor that is mounted around the periphery or other non-treatment areas of the plasma emitter. This grounding conductor is optionally mounted to the tissue via a conductive tissue adhesive or gel. This conductor can be connected to the power supply through a separate connector. As in the previous discussion, it is possible to monitor the connection (and thereby the overall current density) of the patch by sending a small current to the grounding pad(s) and measuring the return current to determine the overall impedance.

The plasma emitter can be connected to the power supply by a variety of techniques. For example, short wires having an external connector may be laminated, glued, soldered, or crimped onto the conductive layer of the flexible plasma emitter. Alternatively, a variety of connectors may be mounted (via soldering, lamination, or gluing) on the conductor of the plasma emitter. These can include, for example, snap connectors, surface mount connectors, pin holes, crimp or clamps connectors, among others. Finally, the conductor of the flexible plasma emitter can be formed into one half of a connector, such as a conductive tab or pin. The plasma emitter can be attached to the treatment area through a variety of fasteners/attachment techniques, including hook and loop fastener, straps, and tissue adhesives. The tissue adhesives may be single-use or multi-use, such as in the case of hydrogels.

<u>EXAMPLES</u>

The following summarizes in vitro work performed by Applicant evidencing that the disclosed systems and techniques are useful for killing the fungus responsible for onychomycosis in clinically-infected nail samples, including work done with a microsecond and a nanosecond power supply.

Applicant recently demonstrated the ability of cold atmospheric pressure plasma to kill *T. rubrum,* the fungus most frequently associated with onychomycosis, in vitro in a simulated nail model.

0.7 x 0.7 cm sections of 317.5 micron thick shim stock were cut and double washed in ethanol for 10 minutes. Next, 1 ml of stock *T. rubrum* was homogenized with 4 ml of sterile SDM (Sabouraud Dextrose media). The sterile shim stock was placed in 5 ml solution of *T. rubrum* and inoculated for 2 hours. After removing the shim stock from solution, it was plasma treated for varying time and frequency settings: 1.5 or 3 kHz, 30 seconds or 3 minutes. The treated shim stock was then placed on an SDA (agar) plate to grow overnight. Experience has suggested that direct treatment of microbes on agar can lead to modification of the agar itself (making it acidic), potentially confounding the results. Hence, the shim stock was inoculated and treated separately prior to placement on fresh agar. Resulting sterilization of the shim stock was determined by visual observation of "growth" or "no growth" as shown in Table 1. We note that in these experiments, the shim stock was still moist/wet with solution prior to treatment with plasma. When the shim stock was allowed to dry after inoculation, the resulting inhibition of fungal growth was less significant (2 - 3 day delay, results not shown). Applicant believes that the plasma treatment in the presence of fluids such as water creates a local acidic environment that can enhance killing efficacy.

TABLE I

|  | Growth | No Growth |
|---|---|---|
| **2 Days Post Treatment** |  |  |
| Control | ✓ |  |
| 1.5 kHz, 30 seconds |  | ✓ |
| 1.5 kHz, 3 minutes |  | ✓ |
| 3 kHz, 30 seconds |  | ✓ |
| 3 kHz, 3 minutes |  | ✓ |
| **9 Days Post Treatment** |  |  |
| Control | ✓ |  |
| 1.5 kHz, 30 seconds |  | ✓ |
| 1.5 kHz, 3 minutes |  | ✓ |
| 3 kHz, 30 seconds |  | ✓ |
| 3 kHz, 3 minutes |  | ✓ |

**Table 1:** Results of in vitro testing of plasma treatment of *T. rubrum.*

In onychomycosis, however, *T. rubrum* and other organisms can often infect the nail bed or infect sections within the nail plate. Like earthworms tunneling through dirt, these fungi can consume the keratin within the nail plate, leaving microscopic pockets within the nail plate. Accordingly, Applicant conducted tests to demonstrate whether atmospheric pressure plasma could be generated within microscopic air gaps of a similar size to those found within nail plates.

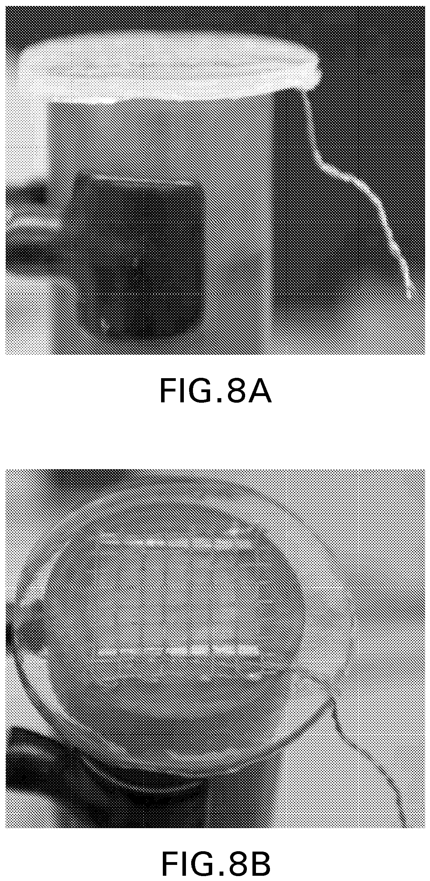

Figures 8a-b respectively illustrate side and top views of an experimental assembly to test generation of plasma within small gaps ranging in size from 12 to 50 microns. The gaps were created using shim stock and glass slides, which were then embedded in gel (to avoid generation of plasma along the periphery). The mesh was connected to ground and used as a transparent electrode to enable visualization of plasma formation within the gap. As shown in Figure 8, two glass slides were epoxied together, separated by two pieces of shim stock having thicknesses of 50, 25 and 12.5 microns. In each case, a high voltage electrode was brought into contact with one side of the glass slides and a mesh was placed on the other side and connected to ground. The entire construction was then embedded in a gel. The mesh was used to enable visualization of the plasma through the glass slide.

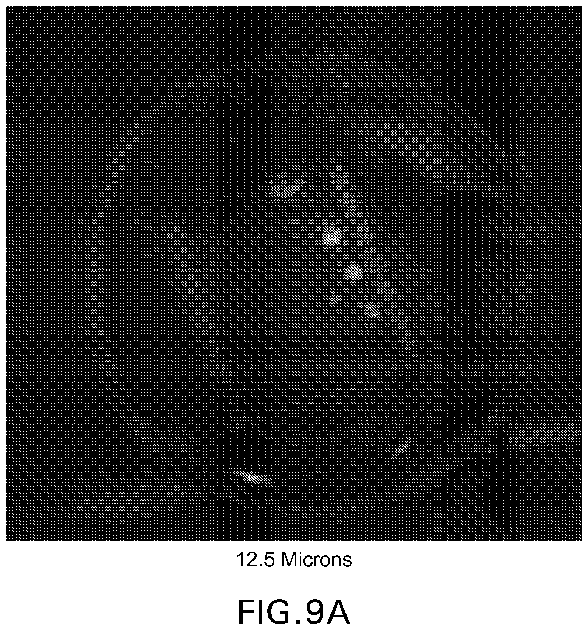

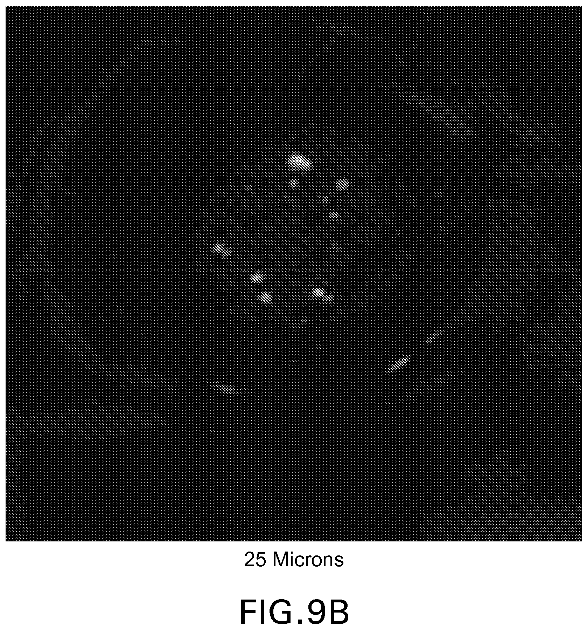

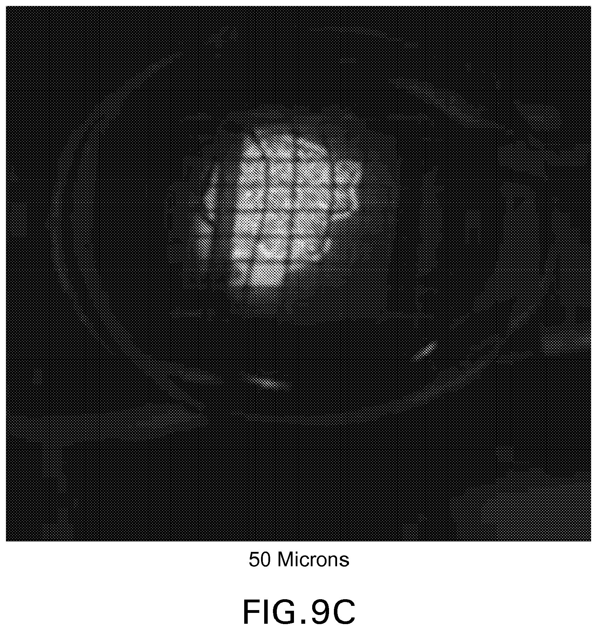

Figure 9 presents photographic evidence of plasma discharge formation within the small gaps at varying thicknesses from about 12.5 to about 50 microns. Applicant notes that the discharge is likely non-uniform due to small differences in the gaps, surface effects or other variations that could cause local concentrations of electric field. These non-uniformities can be easily overcome through appropriate design of the electrodes and power supplies. As shown in Figure 2, plasma was successfully generated within each of the gaps, evidencing that plasmas can by analogy be generated in nail structures that are infected with *T. rubrum* and/or other organisms.

Finally, clinically-infected nail plates were harvested from human volunteers. Control samples were taken, sliced into 50 micron thick slices with a cryotome and cultured, confirming infection throughout the nail plate with *T. rubrum*. The remaining samples were treated with a pulsed dielectric barrier discharge at a frequency of approximately 200 - 300 Hz and a voltage of 20 kV for a duration of 5 minutes. The nail plates were treated three times for this duration. The first time, the nail plate was treated face up and then face down. The third time, the nail plate was embedded in a gel in order to enable generation of plasma within gaps in the nail plate. In all cases, the nail clippings were treated in their "dry" state.

Upon completion of the treatment, the samples were frozen and then sliced into 50 micron thick slices with a cryotome. The slices were then placed on agar and incubated as before. Table 2 below shows the number of days that elapsed until growth was seen on each slice. As shown, nearly all slices in the control sample grew by day 2, whereas the treated samples took an average of 7 days to grow. It is known that vegetative *T. rubrum* populations will double on average every 4.5 hours. Accordingly, this delay in growth represents approximately 4 - 5 log reduction in the fungal population.

## TABLE 2

| slice (microns) | Days of Delayed Growth* 5 minute treatments | | | | |
| --- | --- | --- | --- | --- | --- |
| | Control | #1 | #2 | #3 | #4 |
| 0-50 | 3 | 11 | X | 3 | X |
| 50-100 | 3 | X | 11 | 3 | X |
| 100-150 | 3 | 10 | 7 | 3 | X |
| 150-200 | 2 | 5 | X | 3 | 4 |
| 200-250 | 2 | X | 5 | 4 | 4 |
| 250-300 | 2 | X | 5 | 4 | X |
| 300-350 | 2 | 5 | 4 | 5 | X |
| 350-400 | 2 | 10 | 3 | 5 | 5 |
| 400-450 | 2 | X | 3 | 5 | 5 |
| 450-500 | 2 | X | 3 | 5 | 5 |
| 500-550 | 2 | X | X | 4 | 4 |
| 550-600 | 2 | X | X | 3 | 4 |
| 600-650 | 2 | 7 | X | 2 | 4 |
| 650-700 | 2 | X | X | 3 | 4 |
| 700-750 | 2 | X | 11 | 2 | 4 |
| 750-800 | 2 | X | X | | 2 |
| 800-850 | 2 | X | 5 | | 2 |
| 850-900 | 2 | X | 4 | | 2 |
| 900-950 | 2 | 6 | 3 | | 2 |
| 950-1000 | 2 | 6 | 3 | | 2 |
| **Average** | 2.2 | 7.5 | 5.2 | 3.6 | 3.5 |
| **Adj. Average** | 2.2 | 11.4 | 8.3 | 3.6 | 6.2 |

**Table 2:** Days to observable growth for- control (untreated) and plasma-treated nail clippings (clinically infected with *T. rubrum.* The experiment was monitored for 13 days. The 'X' denotes that no growth was observed after 13 days. The average growth is computed for all samples. The adjusted average growth is calculated based on the assumption that all remaining samples grew on Day 14.

For reference, this fungus is estimated to double in population every 4.5 hours. So, if nothing grew after about 7-8 days, this corresponds to at least 6 log reduction.

Exemplary Plasma Treatment System Overview

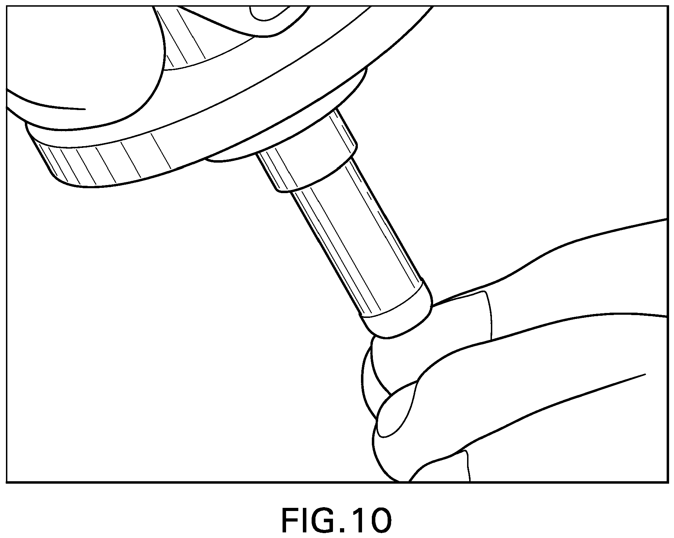

Applicant has developed a technology to deliver atmospheric DBD plasma to toenails with a convenient disposable electrode. The Plasma Pin™ device is applied to the tissue (e.g., nail) and connected to a high voltage power supply and control system, as shown in Figure 10. This power supply generates the electric field characteristics within the patch that are necessary to initiate the formation of non-thermal DBD plasma proximate to the nail and within the nail. The control system has settings to enable the user to vary the intensity of the plasma as desired. Upon activation, plasma forms as a result of the electric field generated between the pin and the nail plate or nail bed below the contact point. It forms along the surface of the dielectric (silicone in this embodiment) and travels down towards the nail plate. Based on laboratory studies, Applicant believes that the plasma also forms within air pockets that may be within the nail plate or nail bed.

In the resulting non-thermal plasma, the plasma serves as a conductor, but at relatively low current is much lower (< 1 milliamp) and, as described above, this non-thermal plasma can be operated in a regime where no damage to tissue (e.g., nail) is observed even over treatment times as long as 20 minutes. Moreover, this current is distributed over a large area (e.g., about 27 sq. mm). By virtue of this moderate local current density, it is expected that the intensity and severity of any side effects will be moderate to the extent that they occur.

As to treatment steps for applying the plasma to a subject, it is contemplated that the following steps can be used:

1. Clean the target treatment site (e.g., nail) for treatment with isopropyl alcohol and allow to dry.
2. Connect the plasma emitter device to its power supply.
3. Connect a Grounding Pad (if provided) to the Ground wire of the Power Supply.
4. Apply the Grounding Pad to skin next to the tissue (e.g., toenail) to be treated.
5. Apply "Dry" Treatment:

- Verify connections from plasma emitter device and ground pad to control system.
- Set controls for frequency and amplitude to desired levels for "Dry" Treatment according to Table 3 below
- Verify trigger is set to 'External'
- Turn system on.
- Set timer to desired duration for Treatment 'Dry' according to Table 3
- Position plasma emitter device in contact with the infected nail plate - light hand pressure is sufficient.
- Flip trigger switch to 'Internal' and activate timer.
- Starting in one of the proximal corners of the nail plate, scan the plasma emitter device in a serpentine path over the entire nail plate. Repeat scanning until treatment duration is complete.
- Once treatment has been completed, flip trigger switch to 'External'

6. Apply a "Wet" treatment by moistening the site (e.g., toenail site) with water.
7. Repeat step 5 for Treatment 'Wet' using the parameters listed in Table 3. Subjects will be asked to undergo treatment until it is no longer tolerable (due to pain, for example) or up to a maximum treatment time of 10 minutes. Moisten the target toenail as needed (5 - 10 times during the treatment).
8. Disconnect and remove and store Ground Pad (one for each patient) after the wet treatment.
9. The patient can then be sent home with e.g., anti-fungal cream and instructions for usage in order to help prevent potential new infections from surrounding tinea pedis if present.

TABLE 3

| Group | Frequency Setting | Amplitude Setting | Dry Nail Duration (min.) | Wet Nail Duration (min.) |
|---|---|---|---|---|
| 1 (5 subjects) | 1 0 (1 kHz) | 1 0 (20 kV) | 2.5 | 2.5 |
| 2 (5 subjects) | 1 o (1 kHz) | 1 0 (20 kV) | 5 | 5 |

(continued)

| Group | Frequency Setting | Amplitude Setting | Dry Nail Duration (min.) | Wet Nail Duration (min.) |
|---|---|---|---|---|
| 3 (5 subjects) | 1 o (1 kHz) | 1 0 (20 kV) | 10 | 10 |

It will be appreciated that the dry and wet treatments can be applied exclusively from the other, or in any desired succession. It will be further appreciated that an electric field alone and/or in combination with plasma can be applied to treat the onychomycosis. Applicant performed in vitro testing on nail clippings using wet and dry techniques to gage their relative efficacy. Two nail control clippings were set aside and then three 3 samples each were treated for 5 minutes per sample using the a power supply at two different pulse repetition frequencies (approx. 400, 800 Hz) and using the same applied voltage (20kV). All of these treatments were done dry (i.e., without adding additional water). Then the experiment was repeated (3 samples each, 2 frequencies) with wet nail samples. As is evident from the below table, after eight days, there was no fungal growth observed on any of the wet samples post treatment. The dry samples showed some growth on the lower frequency treatment, but not on the higher frequency. These results are summarized in Table 4 below:

TABLE 4

| | Total = 8 Days | |
|---|---|---|
| | Dry | Wet |
| Control 1 | 2 | 2 |
| Control 2 | 3 | 2 |
| 20kV, 400Hz | 3 | --- |
| 20kV, 400Hz | 3 | --- |
| 20kV, 400Hz | 4 | --- |
| 20kV, 800Hz | --- | --- |
| 20kV, 800Hz | --- | --- |
| 20kV, 800Hz | --- | --- |

Entries in the above table denoting "---" indicate no detected growth after eight days. In order to address various issues and advance the art, the entirety of this application (including the Cover Page, Title, Headings, Field, Background, Summary, Brief Description of the Drawings, Detailed Description, Claims, Abstract, Figures, Appendices and/or otherwise) shows by way of illustration various embodiments in which the claimed inventions may be practiced. The advantages and features of the application are of a representative sample of embodiments only, and are not exhaustive and/or exclusive. They are presented only to assist in understanding and teach the claimed principles. It should be understood that they are not representative of all disclosed embodiments. As such, certain aspects of the disclosure have not been discussed herein. That alternate embodiments may not have been presented for a specific portion of the invention or that further undescribed alternate embodiments may be available for a portion is not to be considered a disclaimer of those alternate embodiments. It will be appreciated that many of those undescribed embodiments incorporate the same principles of the invention and others are equivalent. Thus, it is to be understood that other embodiments may be utilized and functional, logical, organizational, structural and/or topological modifications may be made without departing from the scope and/or spirit of the disclosure. As such, all examples and/or embodiments are deemed to be non-limiting throughout this disclosure. Also, no inference should be drawn regarding those embodiments discussed herein relative to those not discussed herein other than it is as such for purposes of reducing space and repetition. For instance, it is to be understood that the logical and/or topological structure of any combination of any program components (a component collection), other components and/or any present feature sets as described in the figures and/or throughout are not limited to a fixed operating order and/or arrangement, but rather, any disclosed order is exemplary and all equivalents, regardless of order, are contemplated by the disclosure. Furthermore, it is to be understood that such features are not limited to serial execution, but rather, any number of threads, processes, services, servers, and/or the like that may execute asynchronously, concurrently, in parallel, simultaneously, synchronously, and/or the like are contemplated by the disclosure. As such, some of these features may be mutually contradictory, in that they cannot be simultaneously present in a single embodiment. Similarly, some features are applicable to one aspect of the invention, and inapplicable to others. In addition, the disclosure includes other inventions not presently claimed. Applicant reserves all rights in those presently unclaimed inventions including the right to claim such inventions, file additional applications, continuations, continuations in part, divisions, and/or the like thereof. As such, it should be understood that advantages, embodiments, examples, functional, features, logical, organizational, structural, topological, and/or other aspects of the disclosure are not to be considered limitations on the disclosure as defined by the claims or limitations on equivalents to the claims. It is to be understood that, depending on the particular needs and/or characteristics of a MOE™ individual and/or enterprise user, database configuration and/or relational model, data type, data transmission and/or network framework, syntax structure, and/or the like, various embodiments of the MOE™ may be implemented that enable a great deal of flexibility and customization.

All statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Descriptions herein of circuitry and method steps and computer programs represent conceptual embodiments of illustrative circuitry and software embodying the principles of the disclosed embodiments. Thus the functions of the various elements shown and described herein may be provided through the use of dedicated hardware as well as hardware capable of executing software in association with appropriate software as set forth herein.

In the disclosure hereof any element expressed as a means for performing a specified function is intended to encompass any way of performing that function including, for example, a) a combination of circuit elements and associated hardware which perform that function or b) software in any form, including, therefore, firmware, microcode or the like as set forth herein, combined with appropriate circuitry for executing that software to perform the function. Applicant thus regard any means which can provide those functionalities as equivalent to those shown herein.

Similarly, it will be appreciated that the system and process flows described herein represent various processes which may be substantially represented in computer-readable media and so executed by a computer or processor, whether or not such computer or processor is explicitly shown. Moreover, the various processes can be understood as representing not only processing and/or other functions but, alternatively, as blocks of program code that carry out such processing or functions.

The methods, systems, computer programs and mobile devices of the present disclosure, as described above and shown in the drawings, among other things, provide for improved magnetic resonance methods, systems and machine readable programs for carrying out the same. It will be apparent to those skilled in the art that various modifications and variations can be made in the devices, methods, software programs and mobile devices of the present disclosure without departing from the spirit or scope of the disclosure. Thus, it is intended that the present disclosure include modifications and variations that are within the scope of the subject disclosure and equivalents.

The invention may be described by reference to the following numbered paragraphs:-

1. A method of treating a fungal infection, comprising:

a) providing an electrode adapted to be placed proximate an anatomical region of interest having a fungal infection; and
b) applying an electric field to the region of interest to kill the fungal infection.

2. A method of treating a fungal infection, comprising:

a) providing an electrode adapted to be placed proximate an anatomical region of interest having a fungal infection; and
b) applying a plasma to the region of interest to kill the fungal infection.

3. The method of any preceding claim, wherein the fungal infection includes onychomycosis.
4. The method of any preceding claim, wherein the method further includes applying a pulsed voltage waveform to the electrode to generate a plasma proximate the electrode, the pulsed voltage waveform having pulses with durations that are shorter than the time required for the formation of microdischarges between the electrode and the anatomical region of interest.
5. The method of paragraph 4, wherein the waveform has a pulse duration between at least one of (i) about 0.000000010 seconds and about 0.00000010 seconds, (ii) about 0.0000000010 seconds and about 0.000000010 seconds, and (iii) about 0.00000000010 seconds and about 0.0000000010 seconds.
6. The method of paragraph 4, wherein the electrode is flexible or resilient.
7. The method of Claim 4, wherein the power deposited by the plasma on the anatomical region of interest is between about 1.0 milliwatts per square centimeter and about 10.0 watts per square centimeter.

8. The method of paragraph 4, wherein the power deposited by the plasma on the anatomical region of interest is between about 10.0 milliwatts per square centimeter and about 1.0 watts per square centimeter.

9. The method of paragraph 4, wherein the power deposited by the plasma on the anatomical region of interest is between about 100.0 milliwatts per square centimeter and about 0.5 watts per square centimeter.

10. The method of paragraph 4, wherein the anatomical region of interest is exposed to the plasma for between about one tenth of a second and about one hour.

11. The method of paragraph 4, wherein the anatomical region of interest is exposed to the plasma for between about five seconds and about fifteen minutes.

12. The method of paragraph 4, wherein the anatomical region of interest is exposed to the plasma for between about thirty seconds and about ten minutes.

13. The method of paragraph 4, wherein the anatomical region of interest is exposed to the plasma for between about three minutes and about seven minutes.

14. The method of any preceding claim, wherein the anatomical region of interest is not wetted with a beneficial agent during the treatment.

15. The method of paragraphs 1-13, wherein the anatomical region of interest is wetted with a beneficial agent prior to applying plasma or an electric field to the region of interest.

16. The method of paragraph 15, wherein the beneficial agent is water.

17. The method of paragraph 15, wherein the beneficial agent includes at least one material selected from the group consisting of organic materials, gaseous materials, gelatinous materials, liquid materials amino acids, saline, deionized water, and phosphate buffered saline.

18. The method of paragraph 15, wherein plasma or an electric field is applied to the region of interest before being wetted and after being wetted.

19. The method of paragraph 15, wherein plasma or an electric field is applied to the region of interest more than once when the region of interest is wetted.

20. The method of paragraph 1, wherein the infection is on or in an animal.

21. The method of paragraph 1, wherein the infection is on or in a human.

22. The method of paragraph 20 or paragraph 21, wherein the disorder further includes psoriasis or another infection.

23. The method of paragraph 4, wherein the plasma is a corona discharge plasma, a dielectric barrier discharge plasma, a microdischarge plasma, an inductively coupled plasma, a microwave induced plasma, a plasma jet, or a capacitively coupled radio frequency induced plasma.

24. The method of paragraph 4, further comprising controllably flowing a gas proximate the region of interest.

25. The method of paragraph 24, wherein the gas composition and flowrate are selected to accomplish at least one of (i) exposing the region of interest to a desired wavelength spectrum of light, (ii) heating the region of interest, (iii) directing electrical current through the region of interest, (iv) delivering chemical species to the region of interest.

26. The method of any preceding claim, wherein reactive ion chemical species are delivered to the region of interest.

27. The method of paragraph 25, wherein the wavelength spectrum and the intensity of the light are selected to stimulate blood flow to the region of interest.

28. The method of paragraph 27, wherein at least some of the light is in (i) the near-infrared range, (ii) the infrared range, (iii) the ultraviolet range and (iv) the visible range.

29. The method of paragraph 28, wherein at least some of the light is in the UVA range, and the method further includes applying psoralen to the region of interest.

30. The method of any preceding Claim, wherein reactive oxygen species and/or reactive nitrogen species are delivered to the region of interest.

31. The method of paragraph 28, wherein at least some of the light is in the UVB range, and wherein the skin disorder is psoriasis or vitiligo.

32. The method of any preceding Claim, further comprising directing plasma along hair toward the skin in the region of interest.

33. The method of any preceding Claim, further comprising applying a sensitizing material to the region of interest prior to application of plasma to the region of interest.

34. The method of any of paragraphs 2-33, further comprising applying a blocking material to tissue proximate the region of interest to protect the tissue from plasma.

35. The method of any of paragraphs 2-34, wherein the power deposited by the plasma on tissue in the region of interest including the disorder is between about 10.0 milliwatts per square centimeter and about 1.0 watts per square centimeter.

36. The method of any of paragraphs 2-34, wherein the power deposited by the plasma on the tissue in the region of interest including the disorder is between about 100.0 milliwatts per square centimeter and about 0.5 watts per square centimeter.

37. The method of any of paragraphs 2-36, wherein the tissue in the region of interest including the disorder is exposed to the plasma for between about thirty seconds and about ten minutes.

38. The method of any of paragraphs 2-36, wherein the tissue in the region of interest including the disorder is exposed to the plasma for between about three minutes and about seven minutes.

39. A processor-readable computer program stored on a tangible non-transient medium for operating a plasma treatment device including a controller, a power source operably coupled and controlled by the controller, and an electrode in operable communication with the power source and controller, wherein the program comprises instructions to cause the controller to operate the power source to induce a plasma between the electrode and a region of interest.

40. The computer program of paragraph 39, wherein plasma treatment device further includes a controllable gas delivery system for directing gas to the region of interest, and wherein the computer program further includes instructions for controlling the flow of gas to the region of interest.

41. A system for applying a plasma discharge, comprising:

a) a controller;
b) an electrode adapted to be placed proximate an anatomical region of interest, the electrode having an array of conductors adapted and configured to be selectively activated and deactivated by the controller; and
c) a power supply in electrical communication with the electrode and
controller, the power supply being adapted and configured to apply power to the electrode to generate a moving plasma over time in the anatomical region of interest by activating a plurality of conductors in the array of conductors.

42. The system of paragraph 41, wherein the electrode is made from material that is resilient and/or flexible.

43. The system of paragraph 41, wherein the power supply is adapted and configured to apply a pulsed voltage waveform to the electrode to generate a plasma proximate the electrode, the pulsed voltage waveform having pulses with durations that are shorter than the time required for the formation of microdischarges between the electrode and the anatomical region of interest.

44. The system of paragraph 41, wherein the electrode is substantially inflexible.

45. The system of paragraph 43 wherein the waveform has a pulse duration between at least one of (i) about 0.000000010 seconds and about 0.00000010 seconds, (ii) about 0.0000000010 seconds and about 0.000000010 seconds, (iii) about 0.00000000010 seconds and about 0.0000000010 seconds, (iv) about 0.000000001seconds and about 0.001 seconds, and (v) about 0.000001 seconds and about 0.001 seconds.

46. The system of paragraph 41, further including a conducting pad adapted to be disposed at a location remote from the electrode in electrical communication with the electrode through the anatomical region of interest.

47. The system of paragraph 46, wherein the controller adjusts the applied voltage in response to a signal received from the conducting pad to maintain a controlled and substantially uniform power deposition in the anatomical region of interest.

48. The system of paragraph 41, wherein the electrode has a distal tip made from a resilient material.

49. The system of paragraph 48, wherein the distal tip has a rounded distal tip to facilitate formation of a surface plasma thereon.

48. A kit, including a beneficial agent according to paragraph 17 in a dispenser and a treatment tip for a plasma treatment device.

49. The kit of paragraph 48, wherein the dispenser is adapted and configured to dispense beneficial agent onto a nail plate.

50. The kit of paragraph 48, wherein the beneficial agent includes a conductive gel for application to a nail plate.

51. The kit of paragraph 48, wherein the beneficial agent includes a material selected from the group consisting of organic materials, gaseous materials, gelatinous materials, liquid materials amino acids, saline, deionized water, and phosphate buffered saline.

52. A method of treatment, comprising:

providing an electrode adapted to be placed proximate an anatomical region of interest to be treated; and
applying an electric field to the region of interest to treat the anatomical region of interest.

53. The method of paragraph 52, wherein the treatment is performed to treat an infection.

54. The method of any of paragraphs 52-53, wherein the infection includes a fungal infection.

55. The method of paragraph 54, wherein the infection includes at least one of onychomycosis and psoriasis.

56. The method of any of paragraphs 52-55, wherein the method further includes applying a pulsed voltage waveform to the electrode to generate a time varying electric field over the anatomical region of interest, the pulsed voltage waveform having pulses with durations that are shorter than the time required for electrical charges in electrolytes in the tissue to screen the electric field.

57. The method of any of paragraphs 52-56, wherein the pulsed waveform has a pulse duration between at least one of (i) about 0.000000010 seconds and about 0.00000010 seconds, (ii) about 0.0000000010 seconds and about 0.000000010 seconds, and (iii) about 0.00000000010 seconds and about 0.0000000010 seconds.

58. The method of any of paragraphs 52-57, wherein the electrode is flexible or resilient.

59. The method of any of paragraphs 52-58, wherein the power deposited by the electric field in the anatomical region of interest is between about 1.0 milliwatts per square centimeter and about 10.0 watts per square centimeter.

60. The method of any of paragraphs 52-59, wherein the power deposited by the electric field in the anatomical region of interest is between about 10.0 milliwatts per square centimeter and about 1.0 watts per square centimeter.

61. The method of any of paragraphs 52-60, wherein the power deposited by the electric field in the anatomical region of interest is between about 100.0 milliwatts per square centimeter and about 0.5 watts per square centimeter.

62. The method of any of paragraphs 52-61, wherein the anatomical region of interest is exposed to the electric field for between about one tenth of a second and about one hour.

63. The method of any of paragraphs 52-62, wherein the anatomical region of interest is exposed to the electric field for between about five seconds and about fifteen minutes.

64. The method of any of paragraphs 52-63, wherein the anatomical region of interest is exposed to the electric field for between about thirty seconds and about ten minutes.

65. The method of any of paragraphs 52-64, wherein the anatomical region of interest is exposed to the electric field for between about three minutes and about seven minutes.

66. The method of any of paragraphs 52-65, wherein the anatomical region of interest is not wetted with a beneficial agent during the treatment.

67. The method of any of paragraphs 52-66, wherein the anatomical region of interest is wetted with a beneficial agent prior to applying the electric field.

68. The method of paragraph 67, wherein the beneficial agent is water.

69. The method of paragraph 67, wherein the beneficial agent includes at least one material selected from the group consisting of organic materials, gaseous materials, gelatinous materials, liquid materials amino acids, saline, deionized water, and phosphate buffered saline.

70. The method of paragraph 67, wherein the electric field is applied before being wetted and after being wetted.

71. The method of paragraph 67, wherein the electric field is applied more than once when the region of interest is wetted.

72. The method of paragraph 52, wherein the anatomical region of interest is on or in an animal.

73. The method of paragraph 52, wherein the anatomical region of interest is on or in a human.

74. The method of paragraph 52, wherein the electric field has a strength between about 3,000V/mm and 20,000V/mm.

75. The method of paragraph 52, wherein the electric field does not result in substantial formation of plasma in the anatomical region of interest.

76. The method of paragraph 52, wherein the electric field results in no detectable plasma formation in or on the anatomical region of interest.

**Claims**

1. A system for applying a plasma discharge and/or electric field, comprising:

   a controller (4);
   an electrode (3) adapted to be placed proximate an anatomical region of interest, the electrode (3) having an array of conductors; and
   a power supply in electrical communication with the electrode (3) and controller (4), the power supply being adapted and configured to apply power to the electrode (3),
   **characterized in that** the array of conductors are adapted and configured to be selectively activated and deactivated by the controller (4), the power supply being adapted and configured to apply power to the electrode (3) to generate a moving plasma over time in the anatomical region of interest by activating a plurality of conductors in the array of conductors.

2. The system of Claim 1, wherein the electrode (3) is made from material that is resilient and/or flexible.

3. The system of Claim 1, wherein the electrode (3) is substantially inflexible.

4. The system of any one of Claims 1 to 3, wherein the power supply is adapted and configured to apply a pulsed voltage waveform to the electrode (3) to generate a plasma proximate the electrode (3), the pulsed voltage waveform having pulses with durations that are shorter than the time required for the formation of microdischarges between the electrode (3) and the anatomical region of interest.

5. The system of Claim 4 wherein the waveform has a pulse duration between at least one of (i) about 0.000000010 seconds and about 0.00000010 seconds, (ii) about 0.0000000010 seconds and about 0.000000010 seconds, (iii) about 0.00000000010 seconds and about 0.0000000010 seconds, (iv) about 0.000000001 seconds and about 0.001 seconds, and (v) about 0.000001 seconds and about 0.001 seconds.

6. The system of any one of Claims 1 to 5, further including a conducting pad adapted to be disposed at a location remote from the electrode (3) in electrical communication with the electrode (3) through the anatomical region of interest.

7. The system of Claim 6, wherein the controller (4) adjusts the applied voltage in response to a signal received from the conducting pad to maintain a controlled and substantially uniform power deposition in the anatomical region of interest.

8. The system of any one of Claims 1 to 7, wherein the electrode (3) has a distal tip made from a resilient material.

9. The system of Claim 8, wherein the distal tip has a rounded distal tip to facilitate formation of a surface plasma thereon.

10. The system of any one of Claims 1 to 9, including a beneficial agent in a dispenser and a treatment tip for a plasma treatment device.

11. The system of Claim 10, wherein the dispenser is adapted and configured to dispense beneficial agent onto a nail plate.

12. The system of Claim 10 or claim 11, wherein the beneficial agent includes a conductive gel for application to a nail plate.

13. The system of any one of Claims 10 to 12, wherein the beneficial agent includes a material selected from the group consisting of organic materials, gaseous materials, gelatinous materials, liquid materials amino acids, saline, deionized water, and phosphate buffered saline.

14. The system of any one of the preceding claims, wherein the electrode is adapted to be disposed on one or more toe nail plates, and further wherein the power supply is adapted to provided a pulsed waveform having a pulse duration on the order of a nanosecond.

15. The system of any one of the preceding claims, further comprising a grounding pad connected to a ground wire of the power supply, the grounding pad being adapted to be applied to skin next to a toenail to be treated using the system.

FIG.1

FIG.2

FIG.3

FIG.4

FIG.5

FIG.6

FIG.7

FIG.8A

FIG.8B

12.5 Microns

FIG.9A

25 Microns

# FIG.9B

50 Microns

FIG.9C

FIG.10

Europäisches
Patentamt

European
Patent Office

Office européen
des brevets

## EUROPEAN SEARCH REPORT

Application Number

EP 17 15 8902

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/022403 A2 (UNIV LELAND STANFORD JUNIOR [US]; CHALBERG THOMAS W JR [US]; BLUMENKRA) 22 February 2007 (2007-02-22)<br>* abstract; claims 1-25; figures 3, 5 *<br>* pages 3-11, 22 * | 1-15 | INV.<br>A61N1/18<br>A61N1/40<br>A61B18/04<br>H05H1/24<br>A61N1/04 |
| X | US 2010/145253 A1 (GUTSOL ALEXANDER F [US] ET AL) 10 June 2010 (2010-06-10)<br>* abstract; claim *; figure * *<br>* paragraphs [0012] - [0018] *<br>* paragraphs [0043] - [0079] * | 1 | A61N1/32<br>A61N1/44<br>A61N5/06<br>A61N1/06 |
| A | US 2008/045879 A1 (PRAUSNITZ MARK R [US] ET AL) 21 February 2008 (2008-02-21)<br>* the whole document * | 1-15 | |
| A | US 2006/189976 A1 (KARNI ZIV [IL] ET AL) 24 August 2006 (2006-08-24)<br>* the whole document * | 1-15 | |
| X | AYAN H ET AL: "Application of nanosecond-pulsed dielectric barrier discharge for biomedical treatment of topographically non-uniform surfaces; Application of nanosecond-pulsed DBD for biomedical treatment of topographically non-uniform surfaces", JOURNAL OF PHYSICS D: APPLIED PHYSICS, INSTITUTE OF PHYSICS PUBLISHING LTD, GB, vol. 42, no. 12, 21 June 2009 (2009-06-21), page 125202, XP020158376, ISSN: 0022-3727<br>* abstract *<br>* pages 1-5 * | 1-15 | TECHNICAL FIELDS SEARCHED (IPC)<br>A61N<br>A61B<br>H05H |

-/--

The present search report has been drawn up for all claims

| Place of search | Date of completion of the search | Examiner |
|---|---|---|
| Munich | 21 June 2017 | Scheffler, Arnaud |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt

European Patent Office

Office européen des brevets

**EUROPEAN SEARCH REPORT**

Application Number

EP 17 15 8902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KUCHENBECKER M ET AL: "Characterization of DBD plasma source for biomedical applications", JOURNAL OF PHYSICS D: APPLIED PHYSICS, INSTITUTE OF PHYSICS PUBLISHING LTD, GB, vol. 42, no. 4, 21 February 2009 (2009-02-21), page 45212, XP020149191, ISSN: 0022-3727, DOI: 10.1088/0022-3727/42/4/045212 * abstract * * pages 1-10 * | 1-15 | |
| A | US 7 758 537 B1 (BRUNELL STEPHEN M [US] ET AL) 20 July 2010 (2010-07-20) * the whole document * | 1-15 | |
| A | US 2010/261994 A1 (DAVALOS RAFAEL [US] ET AL) 14 October 2010 (2010-10-14) * the whole document * | 1-15 | |
| | | | TECHNICAL FIELDS SEARCHED (IPC) |

The present search report has been drawn up for all claims

| Place of search | Date of completion of the search | Examiner |
|---|---|---|
| Munich | 21 June 2017 | Scheffler, Arnaud |

page 2 of 2

## ANNEX TO THE EUROPEAN SEARCH REPORT
## ON EUROPEAN PATENT APPLICATION NO.

EP 17 15 8902

This annex lists the patent family members relating to the patent documents cited in  the above-mentioned European search report.
The members are as contained in the European Patent Office EDP file on
The European Patent Office is in no way liable for these particulars which are merely given for the purpose of information.

21-06-2017

| Patent document cited in search report | | Publication date | Patent family member(s) | | Publication date |
|---|---|---|---|---|---|
| WO 2007022403 | A2 | 22-02-2007 | AU 2006279395 A1<br>CA 2620294 A1<br>EP 1924698 A2<br>JP 2009507780 A<br>WO 2007022403 A2 | | 22-02-2007<br>22-02-2007<br>28-05-2008<br>26-02-2009<br>22-02-2007 |
| US 2010145253 | A1 | 10-06-2010 | AU 2006239843 A1<br>CA 2605650 A1<br>EP 1876986 A2<br>JP 2008539007 A<br>US 2010145253 A1<br>US 2013310731 A1<br>WO 2006116252 A2 | | 02-11-2006<br>02-11-2006<br>16-01-2008<br>13-11-2008<br>10-06-2010<br>21-11-2013<br>02-11-2006 |
| US 2008045879 | A1 | 21-02-2008 | EP 1755733 A2<br>US 2008045879 A1<br>WO 2006004595 A2 | | 28-02-2007<br>21-02-2008<br>12-01-2006 |
| US 2006189976 | A1 | 24-08-2006 | CA 2573522 A1<br>EP 1810626 A2<br>IL 180519 A<br>US 2006189976 A1 | | 18-07-2007<br>25-07-2007<br>27-02-2014<br>24-08-2006 |
| US 7758537 | B1 | 20-07-2010 | AU 4223300 A<br>EP 1178757 A1<br>JP 2002541902 A<br>US 7758537 B1<br>WO 0062685 A1 | | 02-11-2000<br>13-02-2002<br>10-12-2002<br>20-07-2010<br>26-10-2000 |
| US 2010261994 | A1 | 14-10-2010 | US 2010261994 A1<br>WO 2010118387 A1 | | 14-10-2010<br>14-10-2010 |

EPO FORM P0459

For more details about this annex : see Official Journal of the European Patent Office, No. 12/82

**REFERENCES CITED IN THE DESCRIPTION**

**Patent documents cited in the description**

- US 201231923 W
- US 61535986 A
- US 61584399 A
- US 2011046382 W